(19) Europäisches Patentamt / European Patent Office / Office européen des brevets

(11) **EP 4 711 467 A1**

(12) **EUROPEAN PATENT APPLICATION**
published in accordance with Art. 153(4) EPC

(43) Date of publication:
**18.03.2026 Bulletin 2026/12**

(21) Application number: **24819295.7**

(22) Date of filing: **03.06.2024**

(51) International Patent Classification (IPC):
*C12P 1/00* (2006.01)      *B01D 63/02* (2006.01)
*C12M 1/00* (2006.01)      *C12M 3/06* (2006.01)
*C12N 1/02* (2006.01)      *C12P 21/08* (2006.01)

(52) Cooperative Patent Classification (CPC):
**B01D 63/02; C07K 16/00; C12M 1/00; C12M 3/06; C12N 1/02; C12P 1/00**

(86) International application number:
**PCT/JP2024/020228**

(87) International publication number:
**WO 2024/253065 (12.12.2024 Gazette 2024/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **09.06.2023 JP 2023095894**

(71) Applicant: FUJIFILM Corporation
**Tokyo 106-8620 (JP)**

(72) Inventors:
• **HIKICHI, Naomichi**
  **Kanagawa 258-8577 (JP)**

• **TANIGUCHI, Kosuke**
  **Kanagawa 258-8577 (JP)**
• **NAKAGAWA, Yosuke**
  **Kanagawa 258-8577 (JP)**
• **INADA, Atsushi**
  **Kanagawa 258-8577 (JP)**
• **YOSHIDA, Shunichi**
  **Kanagawa 258-8577 (JP)**
• **NAKAI, Shinichi**
  **Kanagawa 258-8577 (JP)**

(74) Representative: Hoffmann Eitle
**Patent- und Rechtsanwälte PartmbB**
**Arabellastraße 30**
**81925 München (DE)**

(54) **PRODUCT PRODUCTION METHOD, HOLLOW FIBER MEMBRANE FILTER AND PRODUCT PRODUCTION DEVICE**

(57)    Provided is a method for manufacturing a product, including a culture step of culturing cells in a culture solution accommodated in a tank having a capacity of 100 L or more, and a separation step of using a hollow fiber membrane filter which has a hollow fiber membrane having a primary flow passage extending in a longitudinal direction inside the hollow fiber membrane and has a housing accommodating a plurality of the hollow fiber membranes, the hollow fiber membrane filter having a secondary flow passage in a space of the housing outside each of the hollow fiber membranes, to feed the culture solution supplied from the tank to the primary flow passage, and allowing a permeated liquid containing the product to permeate through the secondary flow passage to separate the cells and the product in the culture solution from each other, in which, in the separation step, in a case where a difference in pressure between both ends of the primary flow passage on a supply side and a discharge side during the feeding of the culture solution is denoted as $\Delta P$ [Pa], and a viscosity of the culture solution is denoted as $\mu$ [Pa·s], $\Delta P/\mu$ during the feeding of the culture solution is $4.9 \times 10^6$ [1/s] or less.

EP 4 711 467 A1

# FIG. 1

# EP 4 711 467 A1

**Description**

BACKGROUND OF THE INVENTION

1. Field of the Invention

The present disclosure relates to a method for manufacturing a product obtained by culturing cells, a hollow fiber membrane filter used in the method for manufacturing a product, and an apparatus for manufacturing a product.

2. Description of the Related Art

For manufacturing a biopharmaceutical or the like, a method of collecting a product contained in a cell culture solution by filtration has been known. As the filtration method, a tangential flow filtration (TFF) method of feeding the cell culture solution to be filtered in a direction parallel to a surface of a filtration membrane (also referred to as a separation membrane) has been proposed. The TFF method has an advantage of being able to suppress clogging of the filtration membrane as compared with a case of feeding the cell culture solution in a direction perpendicular to the filtration membrane. However, even with the TFF method, the clogging of the filtration membrane occurs due to long-term use.

JP2021-511202A discloses a hollow fiber membrane filter as the filtration membrane used for filtration by the TFF method. JP2021-511202A proposes to use a hollow fiber membrane having an inner diameter of 0.75 mm to 13 mm, in order to increase a shear rate and enhance flushing of cells and cell debris on a membrane wall surface.

JP2018-183150A discloses that a hollow fiber membrane having various inner diameters such as approximately 10,000 $\mu$m, approximately 9,000 $\mu$m, and approximately 8,000 $\mu$m and various lengths such as approximately 100 mm and approximately 200 mm can be applied as a TFF filter.

SUMMARY OF THE INVENTION

The filtration membrane used for filtering the product has a service life because filtration performance gradually decreases due to membrane clogging during use. A hollow fiber membrane filter using the hollow fiber membrane disclosed in JP2021-511202A also has the service life, but a culture period can be extended as the service life is increased.

In the manufacturing of the product, in order to improve manufacturing efficiency, studies on continuous operation in which the culture of the cells and the recovery of the product by filtration are continuously performed in parallel while the culture solution is circulated in a circulation path have been conducted. In this case, in a case where the service life of the hollow fiber membrane filter is short, a continuous operation period is shortened, and thus the manufacturing efficiency of the culture product is decreased. In particular, in a case of scaling up a culture tank, and an amount of a filtered culture solution is large, which is a problem.

The technology of the present disclosure has been made in view of the circumstances described above, and an object thereof is to extend a continuous operation period of culture in a method for manufacturing a product, in which a product is obtained by culturing cells, a hollow fiber membrane filter used in the method for manufacturing a product, and an apparatus for manufacturing a product.

The method for manufacturing a product according to the present disclosure is a method for manufacturing a product obtained by culturing cells, the method comprising:

a culture step of culturing the cells in a culture solution accommodated in a tank having a capacity of 100 L or more; and
a separation step of using a hollow fiber membrane filter which has a hollow fiber membrane having a primary flow passage extending in a longitudinal direction inside the hollow fiber membrane and has a housing accommodating a plurality of the hollow fiber membranes, the hollow fiber membrane filter having a secondary flow passage in a space of the housing outside each of the hollow fiber membranes, to feed the culture solution supplied from the tank to the primary flow passage, and allowing a permeated liquid containing the product to permeate through the secondary flow passage to separate the cells and the product in the culture solution from each other,
in which, in the separation step, in a case where a difference in pressure between both ends of the primary flow passage on a supply side and a discharge side during the feeding of the culture solution is denoted as $\Delta P$ [Pa], and a viscosity of the culture solution is denoted as $\mu$ [Pa·s], $\Delta P/\mu$ during the feeding of the culture solution is $4.9 \times 10^6$ [1/s] or less.

It is preferable that a relationship between a length L of the hollow fiber membrane and an inner diameter d of the hollow fiber membrane satisfies $L/d \leq 290$.

It is preferable that a relationship between a total cross-sectional area S1 of the primary flow passage in a cross section perpendicular to a length direction of the hollow fiber membrane filter and a cross-sectional area Sh of the housing satisfies S1/Sh ≥ 0.28.

It is preferable that a membrane resistance r of the hollow fiber membrane is 1,250 psi/mL/min or more.

It is preferable that a pressure loss coefficient k between the primary flow passage and the secondary flow passage of the hollow fiber membrane is $5 \times 10^{13}/m^2$ or more.

It is preferable that a relationship between an outer diameter D and an inner diameter d of the hollow fiber membrane satisfies D/d ≥ 1.1.

It is preferable that a surface roughness Sa of a wall surface of the primary flow passage of the hollow fiber membrane is 8 μm or less.

It is preferable that, in the separation step, a direction of the feeding is reversed at a preset timing.

It is preferable that the product is an antibody.

It is preferable that the method for manufacturing a product according to the present disclosure is a perfusion culture including a step of recovering the product and a supply step of supplying a culture medium to the tank.

It is preferable that a cell density of the culture solution is $30 \times 10^6$ cells/mL or more.

It is preferable that a titer of the product is 1.0 g/L or more.

The hollow fiber membrane filter according to the present disclosure is a hollow fiber membrane filter used for a method for manufacturing a product obtained by culturing cells in a culture solution accommodated in a tank having a capacity of 100 L or more, the hollow fiber membrane filter comprising:

a hollow fiber membrane having a primary flow passage extending in a longitudinal direction inside the hollow fiber membrane; and
a housing accommodating a plurality of the hollow fiber membranes,
in which a length L of the hollow fiber membrane and an inner diameter d of the hollow fiber membrane each satisfy L ≤ 290 mm and d ≥ 1 mm, and
a relationship between the length L and the inner diameter d satisfies L/d ≤ 290.

It is preferable that a product N × D of a number N of the hollow fiber membranes per square centimeter in a cross section perpendicular to a length direction of the housing and an outer diameter D of the hollow fiber membrane is 5 pieces·cm/cm² or more.

It is preferable that a relationship between a total cross-sectional area S1 of the primary flow passage in a cross section perpendicular to a length direction of the hollow fiber membrane filter and a cross-sectional area Sh of the housing satisfies S1/Sh ≥ 0.28.

It is preferable that a pressure loss coefficient k of the hollow fiber membrane is $5 \times 10^{13}/m^2$ or more.

It is preferable that a relationship between an outer diameter D and the inner diameter d of the hollow fiber membrane satisfies D/d ≥ 1.1.

It is preferable that a hydraulic radius reff represented by the following expression using a number M of the hollow fiber membranes accommodated in the housing, an inner diameter $D_h$ of the housing, and an outer diameter D of the hollow fiber membrane is 0.55 or less.

$$\text{reff} = \pi(D_h^2 - M \cdot D^2)/2\pi(D_h + M \cdot D)$$

It is preferable that a contact angle of water on a wall surface of the primary flow passage of the hollow fiber membrane is 75° or less.

The apparatus for manufacturing a product according to the present disclosure is an apparatus for manufacturing a product used for a method for manufacturing a product obtained by culturing cells in a culture solution, the apparatus comprising:

a tank having a capacity of 100 L or more, the tank accommodating the culture solution;
a hollow fiber membrane filter in which a length L of a hollow fiber membrane and an inner diameter d of the hollow fiber membrane each satisfy L ≤ 290 mm and d ≥ 1 mm, and a relationship between the length L and the inner diameter d satisfies L/d ≤ 290;
a first pump which feeds the culture solution to a primary flow passage of the hollow fiber membrane filter; and
a second pump which feeds, to a product recovery part, a permeated liquid passing through a secondary flow passage of the hollow fiber membrane filter in a case where the culture solution is fed along the primary flow passage of the hollow fiber membrane filter.

According to the technology of the present disclosure, it is possible to extend a continuous operation period of culture in a method for manufacturing a product, in which a product is obtained by culturing cells, a hollow fiber membrane filter used in the method for manufacturing a product, and an apparatus for manufacturing a product.

BRIEF DESCRIPTION OF THE DRAWINGS

FIG. 1 is a view showing an overall configuration of a cell culture apparatus.
FIG. 2 is an explanatory view of a hollow fiber membrane filter.
FIG. 3 is a cross-sectional view taken along a line III-III of FIG. 2.
FIG. 4 is a view schematically showing a primary flow passage and a secondary flow passage of the hollow fiber membrane filter.
FIG. 5 is a graph showing a relationship between a treatment time of a separation step and a transmembrane pressure difference, and a schematic view showing a change in a state of a membrane surface of the hollow fiber membrane with the lapse of the treatment time.
FIG. 6 is a schematic view showing a configuration of a simple experimental system.
FIG. 7 is a view showing a flow rate of permeation in a length direction of a hollow fiber membrane 22 in a case (a) where a culture solution is fed forward to the hollow fiber membrane filter and a case (b) where the culture solution is fed backward to the hollow fiber membrane filter.
FIG. 8A is a view for describing a mechanism of occurrence of a bypass flow in a hollow fiber membrane filter having a hollow fiber membrane with a relatively long membrane length and FIG. 8B is a view for describing a mechanism of occurrence of a bypass flow in a hollow fiber membrane filter having a hollow fiber membrane with a relatively short membrane length.

DESCRIPTION OF THE PREFERRED EMBODIMENTS

Hereinafter, the method for manufacturing a product according to the present disclosure will be described. However, an embodiment according to the present disclosure is not limited to the following embodiment, and can be implemented by being appropriately modified.

In the present disclosure, the numerical ranges shown using "to" means ranges including the numerical values described before and after "to" as the minimum value and the maximum value.

Regarding numerical ranges that are described stepwise in the present disclosure, an upper limit value or a lower limit value described in a numerical range may be replaced with an upper limit value or a lower limit value of another stepwise numerical range. In addition, in a numerical range described in the present disclosure, an upper limit or a lower limit described in a certain numerical range may be replaced with a value described in Examples.

In the present disclosure, the term "step" includes not only an independent step but also a step which cannot be clearly distinguished from other steps, as long as the intended purpose of the step is achieved.

Constituent elements indicated by the same reference numeral in the drawings mean the same constituent element.

The method for manufacturing a product according to the present disclosure is a method for manufacturing a product obtained by culturing cells, the method including:

a culture step of culturing the cells in a culture solution accommodated in a tank having a capacity of 100 L or more; and
a separation step of using a hollow fiber membrane filter which has a hollow fiber membrane having a primary flow passage extending in a longitudinal direction inside the hollow fiber membrane and has a housing accommodating a plurality of the hollow fiber membranes, the hollow fiber membrane filter having a secondary flow passage in a space of the housing outside each of the hollow fiber membranes, to feed the culture solution supplied from the tank to the primary flow passage, and allowing a permeated liquid containing the product to permeate through the secondary flow passage to separate the cells and the product in the culture solution from each other,
in which, in the separation step, in a case where a difference in pressure between both ends of the primary flow passage on a supply side and a discharge side during the feeding of the culture solution is denoted as $\Delta P$ [Pa], and a viscosity of the culture solution is denoted as $\mu$ [Pa·s], $\Delta P/\mu$ during the feeding of the culture solution is $4.9 \times 10^6$ [1/s] or less.

The culture solution contains a culture medium, cells, and a product produced by the cells. The cells have, for example, a diameter of 5 $\mu$m or more, and a volume fraction of the cells in the culture solution is, for example, 1% or more.

The cells are not particularly limited, and examples thereof include eukaryotic cells such as animal cells, plant cells, and yeast, and prokaryotic cells such as Bacillus subtilis and Escherichia coli. Animal cells such as Chinese Hamster Ovary (CHO) cells, Baby Hamster Kidney (BHK)-21 cells, Human Embryonic Kidney (HEK) cells, C127 cells, NS0 cells, and SP2/0-Ag14 cells are preferable; and CHO cells are more preferable because they are extensively characterized and have well-established genetic engineering methods. Even in a case where the cells do not originally produce the desired product or produce a small amount of the product, the desired product can be efficiently produced by, for example, introducing an expression vector encoding a necessary protein, such as a plasmid, into the cells.

The product according to the present disclosure is not particularly limited as long as it is a substance produced by the above-described cells in the culture solution, and examples thereof include substances such as alcohol, an antibiotic, and a protein such as an antibody and an enzyme. Among these, the product is preferably a protein and more preferably an antibody.

In a case where the product is, for example, an antibody, the antibody may be produced by the animal cells such as the CHO cells. The antibody to be produced by the animal cells is not particularly limited; and examples thereof include an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody. The antibody includes not only monoclonal antibodies derived from animals such as humans, mice, rats, hamsters, rabbits, and monkeys, but also engineered antibodies such as chimeric, humanized, and bispecific antibodies.

The desired product can be obtained by culturing the cells producing the product.

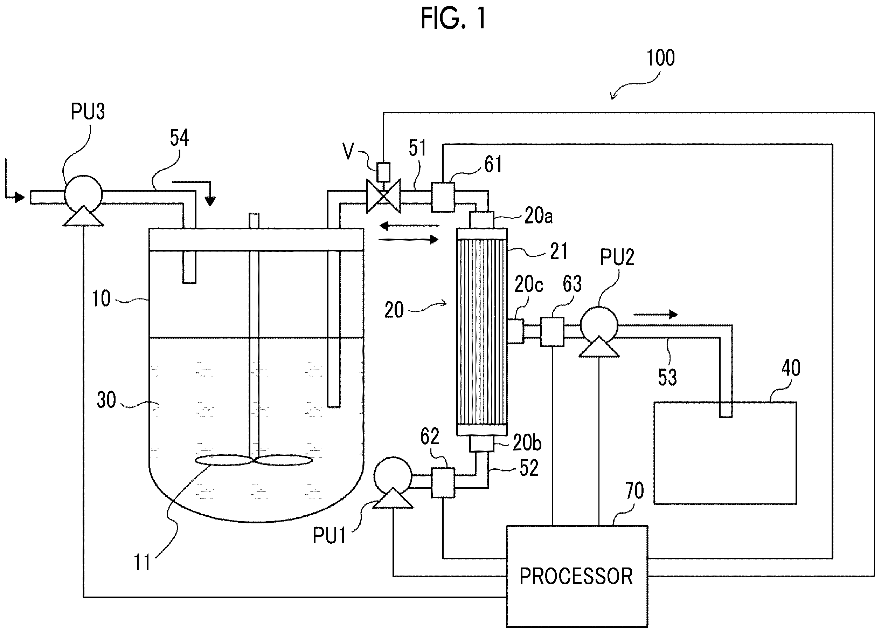

Hereinafter, embodiments of the method for manufacturing a product according to the present disclosure and the apparatus for manufacturing a product, for achieving the method for manufacturing a product, will be described. A cell culture apparatus 100 shown as an example in FIG. 1 is an example of the apparatus for manufacturing a product, which cultures the cells according to the present disclosure to obtain a product. FIG. 1 is a view showing an overall configuration of the cell culture apparatus 100.

As shown in FIG. 1, the cell culture apparatus 100 includes a tank 10, a hollow fiber membrane filter 20, a product recovery part 40, a first pump PU1 to a third pump PU3, flow passages 51 to 54, and a processor 70.

The tank 10 is a culture container which accommodates a culture solution 30 and cultures cells in the culture solution 30. A culture step is performed in the tank 10. The tank 10 has a capacity of 100 L or more. The capacity of the tank 10 is preferably 400 L or more, and more preferably 1,000 L or more. In addition, the capacity of the tank 10 is preferably 200,000 L or less.

A stirring device having a stirring blade 11 is provided inside the tank 10. By rotating the stirring blade 11, the culture solution 30 accommodated in the tank 10 is stirred, and thus homogeneity of the culture solution 30 is maintained.

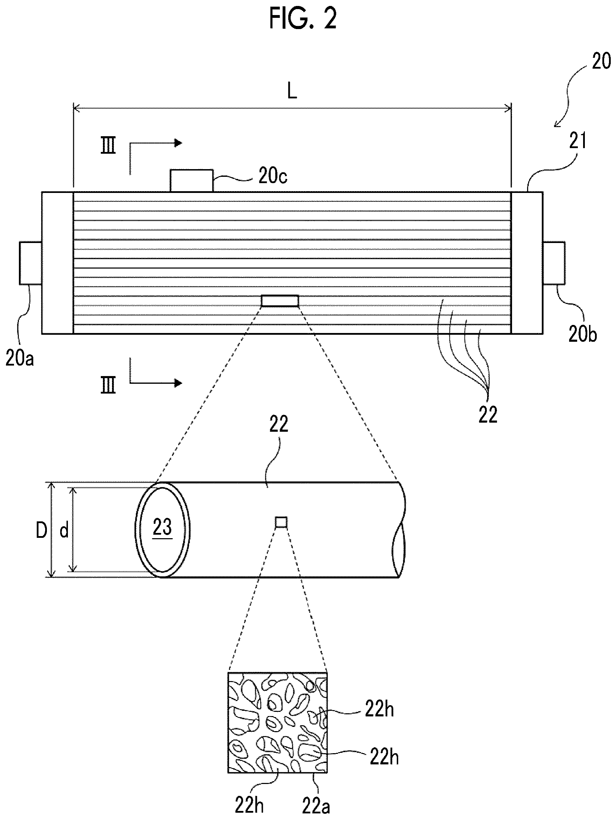

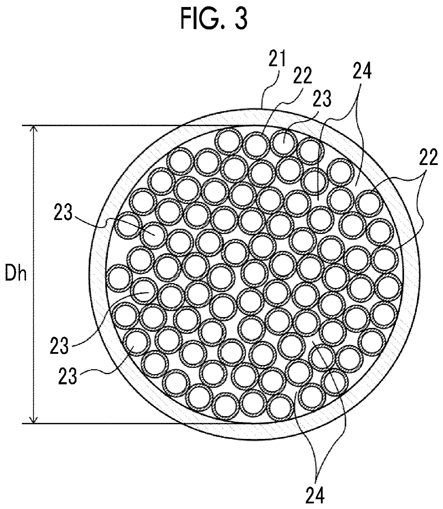

The hollow fiber membrane filter 20 is used in a separation step of separating cells and a product in the culture solution 30. FIG. 2 is a side view showing a schematic configuration of the hollow fiber membrane filter 20; and FIG. 3 is a cross-sectional view taken along a line III-III in FIG. 2.

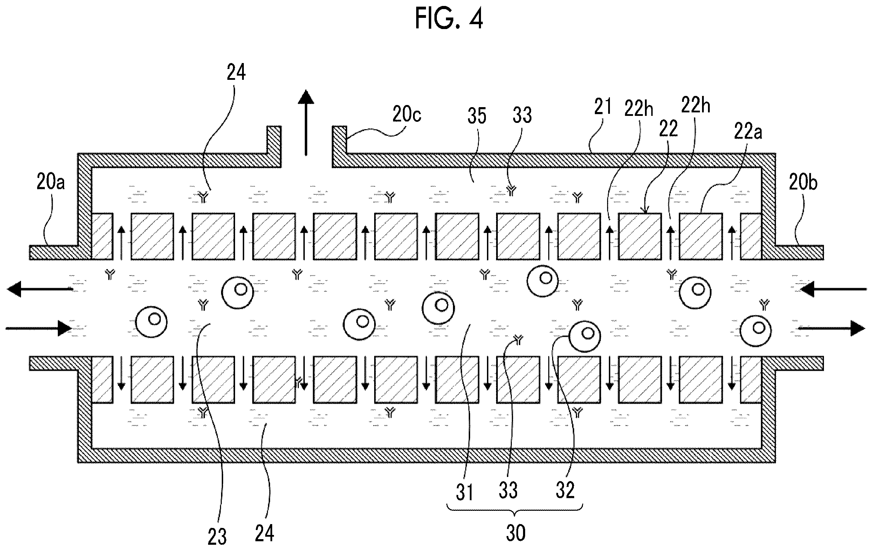

As shown in FIG. 2, the hollow fiber membrane filter 20 includes a hollow fiber membrane 22 having a primary flow passage 23 extending in a longitudinal direction inside the hollow fiber membrane 22, and a housing 21 accommodating a plurality of the hollow fiber membranes 22. The hollow fiber membrane 22 is a separation membrane formed by processing a porous membrane 22a having a large number of pores 22h (see FIG. 1 and FIG. 4 described later) into a tubular shape. A hollow portion of the hollow fiber membrane 22 constitutes the primary flow passage 23. In the hollow fiber membrane filter 20, a space outside each hollow fiber membrane 22 in the housing 21 is a secondary flow passage 24 (see FIG. 3).

The housing 21 has a tubular outer shape, and has, at both ends thereof, a first opening portion 20a and a second opening portion 20b for allowing the culture solution 30 to flow into or out of the primary flow passage 23. In addition, the housing 21 includes a discharge port 20c for discharging a permeated liquid 35 (see FIG. 4) to the outside of the hollow fiber membrane filter 20 on an outer periphery thereof.

FIG. 4 is a view schematically showing the primary flow passage 23 and the secondary flow passage 24 of the hollow fiber membrane filter 20. As shown in FIGS. 2 and 3, in practice, a plurality of the hollow fiber membranes 22 are accommodated in the housing 21; but in FIG. 4, in order to easily understand the function of one hollow fiber membrane 22, an aspect in which one hollow fiber membrane 22 is accommodated in the housing 21 is shown. In the hollow fiber membrane filter 20, the culture solution 30 containing a culture medium 31, cells 32, and an antibody 33 is fed to the primary flow passage 23. The antibody 33 is an example of a product produced by the cells 32. The hollow fiber membrane 22 separates the cells 32 and the antibody 33 in the culture solution 30 by allowing the permeated liquid 35 containing the antibody 33 to permeate through the secondary flow passage 24.

In the hollow fiber membrane 22, among the components in the culture solution 30 passing through the primary flow passage 23, components smaller than the pores 22h of the porous membrane 22a (hereinafter, referred to as pores 22h of the hollow fiber membrane 22) pass through the pores 22h and are transferred to the secondary flow passage 24. That is, the components smaller than the pores 22h of the hollow fiber membrane 22 permeate through the hollow fiber membrane 22. On the other hand, among the components of the culture solution 30 passing through the primary flow passage 23, components larger than the pores 22h remain in the primary flow passage 23. Here, the pores 22h are smaller than the cells 32 and larger than the antibody 33. Since the antibody 33 in the culture solution 30 fed through the primary flow passage 23 of the hollow fiber membrane 22 is smaller than the pores 22h of the hollow fiber membrane 22, the antibody 33 permeates to the secondary flow passage 24 side, whereas the cell 32 is larger than the pores 22h, and thus does not permeate to the secondary flow passage 24 side and remains in the primary flow passage 23. In the separation step, the cells 32 and the antibody 33 are separated using the hollow fiber membrane 22 in this way.

In FIG. 1, the flow passage 51 has one end connected to the tank 10 and the other end connected to the first opening portion 20a of the hollow fiber membrane filter 20 in communication with the primary flow passage 23.

The flow passage 52 has one end connected to the second opening portion 20b of the hollow fiber membrane filter 20 in communication with the primary flow passage 23 and the other end connected to the first pump PU1.

The first pump PU1 has a suction function and a discharge function of the culture solution 30. That is, the first pump PU1 sucks the culture solution 30 from the tank 10 into the hollow fiber membrane filter 20 through the first opening portion 20a, and sucks the culture solution 30 discharged from the second opening portion 20b after passing through the hollow fiber membrane filter 20. In addition, the first pump PU1 discharges the culture solution 30 once sucked, and then supplies the culture solution 30 again to the hollow fiber membrane filter 20 from the second opening portion 20b. That is, the first pump PU1 repeats suction and discharge of the culture solution 30 to reciprocate the culture solution 30 in the hollow fiber membrane filter 20 in a forward direction and a reverse direction. Here, with regard to the feeding direction of the culture solution 30 in the hollow fiber membrane filter 20, a direction from the first opening portion 20a to the second opening portion 20b is defined as the forward direction; and a direction from the second opening portion 20b to the first opening portion 20a is defined as the reverse direction. In addition, hereinafter, a case of feeding the culture solution 30 in the forward direction is referred to as forward feeding, and a case of feeding the culture solution 30 in the reverse direction is referred to as reverse feeding.

The first pump PU1 is, for example, a diaphragm pump, and the culture solution 30 is temporarily stored in a space generated by movement of the diaphragm. The first pump PU1 is used as both a suction port and a jetting port, and thus is capable of forming the reciprocating flow by the movement of the diaphragm.

By the operation of the first pump PU1, the culture solution 30 is fed through the primary flow passage 23 of the hollow fiber membrane 22 in the length direction thereof. That is, the culture solution 30 is fed along a membrane surface of the hollow fiber membrane 22, and a filtration method using a tangential filtration method (TFF method) of allowing a permeated liquid containing a product to permeate a secondary flow passage is adopted. The first pump PU1 reverses the direction of the feeding of the culture solution 30 at a preset timing to form a flow in which the culture solution 30 reciprocates in the hollow fiber membrane filter 20. In a case of reversing the direction of the feeding at a preset timing, for example, the direction of the feeding may be reversed at predetermined time intervals.

In a case of the forward feeding, the culture solution 30 taken out from the tank 10 is supplied from the first opening portion 20a to the primary flow passage 23 of the hollow fiber membrane filter 20 through the flow passage 51. The culture solution 30 passed through the primary flow passage 23 is discharged from the second opening portion 20b, and stored in the first pump PU1 through the flow passage 52. In a case of the reverse feeding, the culture solution 30 stored in the first pump PU1 is supplied from the second opening portion 20b to the primary flow passage 23 of the hollow fiber membrane filter 20 through the flow passage 52. The culture solution 30 passed through the primary flow passage 23 is discharged from the first opening portion 20a, and returned to the tank 10 through the flow passage 51.

As described above, in a case of the forward feeding in which the culture solution 30 is supplied from the tank 10 to the hollow fiber membrane filter 20, the first opening portion 20a on the tank 10 side is a supply side and the second opening portion 20b is a discharge side. On the other hand, in a case of the reverse feeding in which the flow of the culture solution 30 is reversed, the second opening portion 20b is a supply side and the first opening portion 20a is a discharge side. That is, the first opening portion 20a and the second opening portion 20b are switched between the supply side and the discharge side each time the direction of the flow is switched.

A first pressure P1, which is a liquid feeding pressure at the one end of the primary flow passage 23 on the first opening portion 20a side, can be measured by a pressure gauge 61 installed between a pinch valve V provided in the middle of the flow passage 51 and the first opening portion 20a. In addition, a second pressure P2, which is a liquid feeding pressure at the other end of the primary flow passage 23 on the second opening portion 20b side, can be measured by a pressure gauge 62 installed between the first pump PU1 and the second opening portion 20b.

One end of the flow passage 53 is connected to the discharge port 20c provided on the outer periphery of the housing 21 of the hollow fiber membrane filter 20, and the other end thereof is connected to the product recovery part 40. The flow passage 53 communicates with the secondary flow passage 24 of the hollow fiber membrane filter 20 through the discharge port 20c.

The second pump PU2 is provided in the flow passage 53, and recovers the permeated liquid 35 from the secondary flow passage 24 of the hollow fiber membrane filter 20 and feeds the permeated liquid 35 to the product recovery part 40. The second pump PU2 is a suction pump, and operates during the forward feeding and the reverse feeding of the culture solution 30. By operating the second pump PU2, a part of the culture solution 30 supplied into the primary flow passage 23 of the hollow fiber membrane filter 20 is suctioned from the primary flow passage 23 to the secondary flow passage 24 side. By the suction, the permeated liquid 35 containing the antibody 33, which has permeated to the secondary flow passage 24 side through the hollow fiber membrane 22, is discharged from the discharge port 20c to the outside of the hollow fiber membrane filter 20, and recovered by the product recovery part 40. A third pressure P3, which is a liquid feeding pressure of the secondary flow passage 24, can be measured by a pressure gauge 63 provided between the discharge port 20c and the second pump PU2.

The antibody 33 contained in the permeated liquid 35 discharged from the secondary flow passage 24 is supplied to the product recovery part 40 including a recovery tank, a purification treatment part which purifies the antibody 33, and the like, through the flow passage 53.

The flow passage 54 is a flow passage for supplying a fresh culture medium, which is unused fresh culture medium, to the tank 10. The third pump PU3 is provided in the middle of the flow passage 54, and feeds the fresh culture medium from a culture medium housing part (not shown) to the tank 10. In a case where the third pump PU3 is operated, the fresh culture medium is added to the tank 10 through the flow passage 54 to compensate for the amount of liquid lost as the permeated liquid.

The processor 70 controls the overall operation of the cell culture apparatus 100. The configuration of the processor 70 is not particularly limited, but for example, the processor 70 is configured with a central processing unit (CPU), a memory such as a non-volatile memory (NVM) and a random access memory (RAM), and the like.

In a case where a pressure difference, which is a difference between pressures at both ends of the primary flow passage 23 on the supply side and the discharge side during the feeding of the culture solution 30, is denoted as $\Delta P$ [Pa], the processor 70 controls, for example, the pressure difference $\Delta P$ [Pa] to satisfy the following conditional expression in the separation step. The condition is a condition in which, in a case where a viscosity of the culture solution 30 is denoted as $\mu$ [Pa·s], a ratio of the pressure difference $\Delta P$ to the viscosity $\mu$ is set to equal to or less than a target value, and specifically, $\Delta P/\mu \leq 4.9 \times 10^6$ [1/s]. The value of the viscosity $\mu$ of the culture solution and the target value of $\Delta P/\mu$ ($4.9 \times 10^6$ [1/s] or less in the present example) are stored in advance in the memory. The processor 70 controls the output of the first pump PU1 and the second pump PU2, the opening degree of the pinch valve V, and the like such that $\Delta P/\mu$ is $4.9 \times 10^6$ [1/s] or less. $\Delta P/\mu$ is more preferably $2.8 \times 10^6$ [1/s] or less, and still more preferably $2.1 \times 10^6$ [1/s] or less. In a case where the viscosity $\mu$ is known, the known value is stored in the memory. On the other hand, in a case where the viscosity $\mu$ fluctuates during the operation period due to a fluctuation factor such as a concentration of the cells 32 contained in the culture solution 30, a relationship expression between the fluctuation factor and the viscosity $\mu$ may be stored in the memory, and the processor 70 may calculate the viscosity $\mu$ according to the fluctuation factor.

As described above, the liquid feeding pressure on the first opening portion 20a side is the first pressure P1, and the liquid feeding pressure on the second opening portion 20b side is the second pressure P2. In a case of the forward feeding, with regard to the pressures at both ends of the primary flow passage 23 on the supply side and the discharge side, the first pressure P1 is the liquid feeding pressure on the supply side and the second pressure P2 is the liquid feeding pressure on the discharge side. On the contrary, in a case of the reverse feeding, the second pressure P2 is the liquid feeding pressure on the supply side and the first pressure P is the liquid feeding pressure on the discharge side. Therefore, the pressure difference $\Delta P$ at both ends is an absolute value of a difference between the first pressure P1 and the second pressure P2, and is defined as $\Delta P = |P1 - P2|$ in consideration of the change in the reference numeral due to the liquid feeding direction. The first pressure P1 and the second pressure P2 can be measured by the pressure gauges 61 and 62 provided in the flow passage, here, the flow passage 51 connected to the first opening portion 20a or the flow passage 52 connected to the second opening portion 20b.

The viscosity $\mu$ [Pa·s] of the culture solution 30 changes depending on the type of the culture medium 31 and the concentration of the cells in the culture solution 30. The viscosity of the culture solution 30 is, for example, 0.5 to 5 mPa·s. The viscosity of the culture solution 30 is measured by temperature-regulating the sampled culture solution at 38°C and using a vibration type viscometer. As the vibration type viscometer, for example, VM-10A manufactured by SEKONIC CORPORATION is used.

Alternatively, in a case where a membrane resistance of the hollow fiber membrane 22 is denoted as r represented by Expression 1 and a flow passage resistance of the secondary flow passage 24 is denoted as R represented by Expression 2, the processor 70 may control the output of the first pump PU1 and the second pump PU2, the opening degree of the pinch valve V, and the like such that a relationship between $\Delta P$, r, and R during the feeding of the culture solution 30 in the separation step is $\Delta P/2(r + R) \leq 0.0005$ mL/min.

$$r = \mu k \cdot \ln(D/d)/2\pi L \qquad \text{Expression 1}$$

$$R = 8\mu LM/\pi A(\text{reff})^2 \qquad \text{Expression 2}$$

Here, k is a pressure loss coefficient of the hollow fiber membrane 22, D is an outer diameter of the hollow fiber membrane 22, d is an inner diameter of the hollow fiber membrane 22, L is a length of the hollow fiber membrane 22, M is the number of the hollow fiber membranes 22 accommodated in the housing 21, reff is a hydraulic radius of the hollow fiber membrane filter 20 represented by Expression 3, and A is a cross-sectional area of the secondary flow passage 24 in a cross section perpendicular to the length direction of the hollow fiber membrane filter 20.

$$\mathrm{reff} = \pi(D_h{}^2 - M\cdot D^2)/2\pi(D_h + M\cdot D) \quad \text{Expression 3}$$

As shown in FIG. 4, in a case where the culture solution 30 is supplied to the primary flow passage 23 of the hollow fiber membrane filter 20 and fed through the primary flow passage 23 along the membrane surface of the hollow fiber membrane 22, the antibody 33 smaller than the pores 22h of the hollow fiber membrane 22 passes through the pores 22h and transferred to the secondary flow passage 24. The first pump PU1 generates a flow in a direction along a membrane surface of the primary flow passage 23 indicated by an arrow along the primary flow passage 23 in FIG. 4 (hereinafter, referred to as a tangential flow). In the primary flow passage 23 of the hollow fiber membrane 22, in a case where the culture solution 30 is fed along the primary flow passage 23, a flow toward a wall surface orthogonal to the direction of the tangential flow is generated, and the antibody 33 is transferred to the secondary flow passage 24 by this flow through the pores 22h.

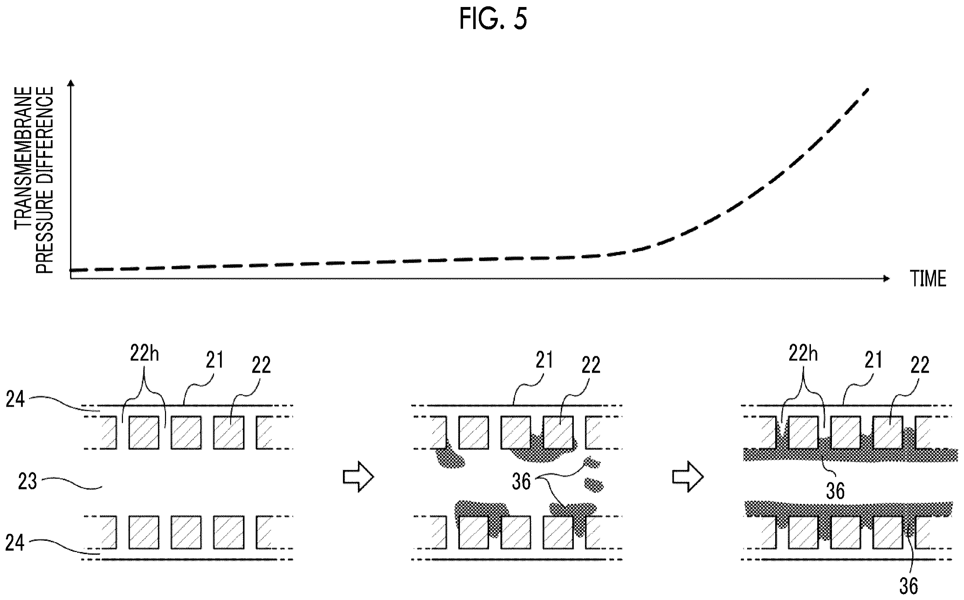

In the primary flow passage 23, the flow toward the membrane surface is generated, and a clogging material having a size which cannot pass through the pores 22h of the hollow fiber membrane 22 enters the pores 22h to cause clogging, or is deposited on the membrane surface. Specifically, the clogging material is an antibody aggregate generated by association and aggregation of hydrophobic portions of the antibody, a dead cell fragment, or the like. Hereinafter, the clogging of the pores 22h by the clogging material and the deposition of the clogging material on the membrane surface are collectively referred to as "membrane clogging". FIG. 5 is a graph showing a relationship between a treatment time of the separation step and a transmembrane pressure difference, and a schematic view showing a change in a state of the membrane surface of the hollow fiber membrane 22 with the lapse of the treatment time.

The transmembrane pressure difference is a difference between an average pressure of the primary flow passage 23 and a liquid feeding pressure of the secondary flow passage 24. The average pressure of the primary flow passage 23 is an average value of liquid feeding pressures at both ends of the primary flow passage 23, that is, an average value of the first pressure P1 at the first opening portion 20a and the second pressure P2 at the second opening portion 20b; and in a case where the average pressure is denoted as P12, P12 = (P1 + P2)/2. The liquid feeding pressure of the secondary flow passage 24 is the third pressure P3 at the discharge port 20c. That is, the transmembrane pressure difference is P12 - P3.

As the transmembrane pressure difference is larger, it is more difficult for a permeation flow to occur from the primary flow passage 23 to the secondary flow passage 24. This is due to the following reason. First, as described above, a part of the culture solution 30 is suctioned from the primary flow passage 23 to the secondary flow passage 24 side by the second pump PU2. Assuming this, a state in which the hollow fiber membrane 22 is not clogged is considered. In a state in which the membrane clogging does not occur, a resistance of the permeation flow permeates through the pores 22h of the hollow fiber membrane 22 to the secondary flow passage 24 side is relatively small, and the permeation flow flows smoothly. Therefore, the transmembrane pressure difference, which is the difference between the average pressure P12 on the primary flow passage 23 side and the third pressure P3 on the secondary flow passage 24 side, is small. On the other hand, in a state in which the membrane clogging occurs, the resistance of the permeation flow passing through the pores 22h of the hollow fiber membrane 22 is large, and the permeation flow is likely to be stagnant. Therefore, the average pressure P12 on the primary flow passage 23 side increases and the third pressure P3 on the secondary flow passage 24 side decreases, and thus the transmembrane pressure difference increases.

FIG. 5 is a graph showing an example of a time change in the transmembrane pressure difference. As shown in FIG. 5, at an initial stage of the separation step, the membrane clogging does not occur and the transmembrane pressure difference is low, but as time elapses, the transmembrane pressure difference gradually increases. As time elapses, the clogging of the pores 22h of the hollow fiber membrane 22 by a clogging material 36 occurs, and finally, the clogging due to the clogging material 36 and the membrane clogging due to the deposition on the membrane surface progress. In a case where the membrane clogging progresses, the separation function of the hollow fiber membrane 22 is reduced, and thus a service life ends. On the contrary, in a case where the progress of the membrane clogging can be delayed, the service life of the hollow fiber membrane 22 can be extended.

As a result of analyzing the flow of the culture solution 30 passing through the hollow fiber membrane 22 and the mechanism of the membrane clogging of the membrane described later, the present inventors have found that the following measures are effective as measures for delaying the progress of the membrane clogging. That is to reduce a proportion of a liquid (bypass liquid described later) other than a liquid (filtrate described later) to be collected in the product recovery part 40 in the permeated liquid permeating from the primary flow passage 23 to the secondary flow passage 24 of the hollow fiber membrane 22.

First, as described above, the membrane clogging occurs because the clogging material having a size larger than the pores 22h is pressed against the membrane surface by the flow from the primary flow passage 23 toward the secondary flow passage 24. The flow of pressing the clogging material against the membrane surface is proportional to a flow rate of the permeated liquid flowing from the primary flow passage 23 to the secondary flow passage 24 through the membrane surface. The flow from the primary flow passage 23 to the secondary flow passage 24 is mainly caused by the operation of the second pump PU2 connected to the secondary flow passage 24, which causes the secondary flow passage 24 to be at a negative pressure with respect to the primary flow passage 23.

In addition, in a case where the permeated liquid permeating from the primary flow passage 23 to the secondary flow passage 24 of the hollow fiber membrane filter 20 is analyzed, it is found that the permeated liquid includes a bypass liquid which is not collected as a filtrate and returns from the secondary flow passage 24 to the primary flow passage 23. Here, in the permeated liquid permeating from the primary flow passage 23 to the secondary flow passage 24, the filtrate is a liquid which is discharged to the outside of the hollow fiber membrane filter 20 and collected in the product recovery part 40. Hereinafter, the flow rate of each of the permeated liquid, the filtrate, and the bypass liquid flowing per unit time to the unit area of the membrane surface of the hollow fiber membrane 22 is referred to as a flow rate of permeation, a flow rate of filtration, and a flow rate of bypass, respectively.

As described above, the membrane clogging progresses as the flow rate of permeation increases. In the flow rate of permeation, the flow rate of filtration cannot be reduced in order to maintain a recovery efficiency of the antibody 33, but the flow rate of bypass does not contribute to the recovery of the antibody 33. Therefore, in a case where the flow rate of bypass can be suppressed (that is, in a case where the proportion of the liquid (bypass liquid described later) other than the liquid (filtrate described later) collected in the product recovery part 40 of the permeated liquid can be reduced), the flow rate of permeation per unit time can be suppressed by the amount of suppression of the flow rate of bypass. As a result of suppressing the flow rate of permeation per unit time, the amount of the clogging material pressed against the wall surface side per unit time can be suppressed, and thus the progress of the membrane clogging can be delayed. As a result, the long-term service life of the hollow fiber membrane filter 20 is achieved.

Hereinafter, a ratio of the flow rate of bypass to the flow rate of permeation, including the flow rate of filtration and the flow rate of bypass, is referred to as a ratio of the flow rate of bypass. Suppressing the flow rate of bypass means reducing the ratio of the flow rate of bypass.

The conditional expression $\Delta P/\mu \leq 4.9 \times 10^6$ [1/s], which is the conditional expression of the ratio of the pressure difference $\Delta P$ and the viscosity $\mu$ described above, is a conditional expression derived based on experiments and the like, on the condition that the tank having a capacity of 100 L or more is used as the tank 10, in order to obtain the service life of the hollow fiber membrane filter 20 corresponding to the capacity. By satisfying the conditional expression, the effect of reducing the ratio of the flow rate of bypass, corresponding to the above-described capacity, is obtained.

In addition, the effect of reducing the ratio of the flow rate of bypass corresponding to the capacity of 100 L is also obtained by satisfying the following condition, instead of or in addition to the condition of $\Delta P/\mu \leq 4.9 \times 10^6$ [1/s]. That is, in the separation step, the condition is that $\Delta P/2(r + R)$ is 0.0005 mL/min or less in a case of feeding the culture solution 30.

As described above, with the method for manufacturing a product according to the present disclosure, the service life of the hollow fiber membrane filter 20 can be extended by reducing the ratio of the flow rate of bypass, and as a result, the culture can be continuously operated for a long period of time.

In the method for manufacturing a product, in which cells are cultured in the culture solution 30 accommodated in the tank 10 having a capacity of 100 L or more to obtain a product, it is preferable to use the following hollow fiber membrane filter 20.

It is preferable that a relationship between a length L and an inner diameter d (see FIG. 2) of the hollow fiber membrane 22 satisfies L/d ≤ 290.

L/d is more preferably 220 or less, still more preferably 200 or less, and even more preferably 140 or less. L/d is preferably 50 or more.

By setting L/d to 290 or less, the pressure difference $\Delta P$ at both ends of the primary flow passage 23 of the hollow fiber membrane 22 can be reduced, and thus $\Delta P/\mu \leq 4.9 \times 10^6$ [1/s] or $\Delta P/2(r + R) \leq 0.0005$ mL/min can be achieved.

As an example, the length L and the inner diameter d of the hollow fiber membrane 22 are L ≤ 290 mm and d ≥ 1 mm. The length L of the hollow fiber membrane is preferably 290 mm or less, more preferably 240 mm or less, and still more preferably 200 mm or less. In addition, the length L of the hollow fiber membrane is preferably 100 mm or more, more preferably 140 mm or more, and still more preferably 150 mm or more. The inner diameter d of the hollow fiber membrane is preferably 1 mm or more, more preferably 1.2 mm or more, still more preferably 1.5 mm or more, and even more preferably 2 mm or more. In addition, the inner diameter d is preferably 5 mm or less, more preferably 4 mm or less, and still more preferably 3 mm or less.

It is preferable that a relationship between a total cross-sectional area S1 of the primary flow passage 23 in a cross section perpendicular to a length direction of the hollow fiber membrane filter 20 and a cross-sectional area Sh of the housing 21 satisfies S1/Sh ≥ 0.28.

Here, the total cross-sectional area S1 of the primary flow passage 23 is a product of a cross-sectional area s1 of one hollow fiber membrane 22 in a cross section perpendicular to the length direction and the number N of the hollow fiber membranes 22 accommodated in the housing 21, and is simply referred to as a primary flow passage cross-sectional area S1 below. S1/Sh is more preferably 0.3 or more, and still more preferably 0.35 or more. S1/Sh is preferably 0.4 or less.

The cross-sectional area Sh of the housing 21 is preferably 2,000 mm$^2$ or more, more preferably 2,400 mm$^2$ or more, still more preferably 3,000 mm$^2$ or more, and even more preferably 5,000 mm$^2$ or more. The upper limit thereof is 10,000 mm$^2$, preferably 8,000 mm$^2$.

By setting S1/Sh to 0.28 or less, the pressure difference $\Delta P$ at both ends of the primary flow passage 23 of the hollow fiber membrane 22 can be reduced, and thus $\Delta P/\mu \leq 4.9 \times 10^6$ [1/s] or $\Delta P/2(r + R) \leq 0.0005$ mL/min can be achieved.

It is preferable that a membrane resistance r of the hollow fiber membrane 22 is 1,250 psi/mL/min (= 8,620 kPa/mL/min) or more.

The membrane resistance r is represented by $r = \mu k \cdot \ln(D/d)2\pi L$.

Here, $\mu$ is a viscosity of the culture solution 30, and k is a pressure loss coefficient of the hollow fiber membrane 22. The membrane resistance r is more preferably 1,600 psi/mL/min or more, and still more preferably 2,500 psi/mL/min or more. In addition, r is preferably 8,000 psi/mL/min or less.

In a case where the membrane resistance r is 1,250 psi/mL/min or more, the flow rate of permeation from the primary flow passage 23 to the secondary flow passage 24 can be effectively suppressed, and the flow rate of bypass can be suppressed.

It is preferable that the pressure loss coefficient k is $5 \times 10^{13}$/m$^2$ or more. The pressure loss coefficient k is a coefficient related to the pressure loss between the primary flow passage 23 and the secondary flow passage 24 of the hollow fiber membrane 22, and depends on the material of the porous membrane 22a constituting the hollow fiber membrane 22, the size of the pores 22h, and the membrane thickness. The pressure loss coefficient k is more preferably $5 \times 10^{14}$/m$^2$ or more, and still more preferably $1 \times 10^{15}$/m$^2$ or more. The pressure loss coefficient k is preferably $1 \times 10^{16}$/m$^2$ or less. The pressure loss coefficient k is calculated by measuring the pressure loss in a case where the solvent is caused to flow from the primary flow passage 23 side to the secondary flow passage 24 side, and dividing the value by the viscosity and the flow velocity of the solvent.

As the pressure loss coefficient k is smaller, the permeated liquid from the primary flow passage 23 to the secondary flow passage 24 is more likely to flow. That is, as the pressure loss coefficient k is smaller, the bypass liquid is more likely to occur. In a case where the pressure loss coefficient k is $5 \times 10^{13}$/m$^2$ or more, the flow rate of permeation can be effectively suppressed, and as a result, the flow rate of bypass can be suppressed.

It is preferable that a relationship between an outer diameter D (see FIG. 2) and the inner diameter d of the hollow fiber membrane 22 satisfies $D/d \geq 1.1$. $D/d \geq 1.1$ means that a membrane thickness t (here, 2t = D - d) of the hollow fiber membrane 22 is 5% or more of the inner diameter d. D/d is more preferably 1.25 or more, and still more preferably 1.4 or more. In addition, D/d is preferably 3.0 or less.

The outer diameter D of the hollow fiber membrane 22 is preferably 1.1 mm or more, and more preferably 1.25 mm or more. In addition, the outer diameter D is preferably 1.4 mm or less.

As D/d is larger, the membrane thickness of the hollow fiber membrane is larger, the membrane resistance r is larger, and the flow rate of permeation from the primary flow passage 23 to the secondary flow passage 24 can be effectively suppressed. In a case where $D/d \geq 1.1$, the flow rate of permeation can be effectively suppressed, and as a result, the flow rate of bypass can be suppressed.

It is preferable that a surface roughness Sa of a wall surface of the primary flow passage 23 of the hollow fiber membrane 22 is 8 $\mu$m or less. The surface roughness Sa is more preferably 7 $\mu$m or less, and still more preferably 6 $\mu$m or less. The surface roughness Sa is preferably 3 $\mu$m or more. The surface roughness Sa is measured using a laser microscope. As the laser microscope, for example, VK-X3000 manufactured by KEYENCE CORPORATION is used.

As the surface roughness Sa of the wall surface of the primary flow passage 23 is smaller, the clogging material is less likely to be caught on the wall surface of the hollow fiber membrane 22, and the clogging of the pores 22h and the deposition on the wall surface can be suppressed. In a case where the surface roughness Sa is 8 $\mu$m or less, the effect of suppressing the membrane clogging is high.

In the hollow fiber membrane filter 20, in a case where the outer diameter D of the hollow fiber membrane 22 is less than 2 mm, the number of the hollow fiber membranes 22 in the housing 21 is preferably 900 or more, and more preferably 1,000 or more. The upper limit thereof is 50,000, preferably 30,000. In order to ensure sufficient treatment capacity even in a case where the hollow fiber membrane 22 is shortened, it is preferable to increase the number of the hollow fiber membranes 22.

In a case where the outer diameter D of the hollow fiber membrane is 2 mm or more, the number of the hollow fibers is preferably 100 or more, and more preferably 200 or more. The upper limit thereof is 10,000, preferably 1,000 or less and more preferably 800 or less.

In the hollow fiber membrane filter 20, it is preferable that a product N $\times$ D of the number N of the hollow fiber membranes 22 per square centimeter in a cross section (see FIG. 3) perpendicular to the length direction of the housing 21 and the outer diameter D of the hollow fiber membrane 22 is 5 pieces·cm/cm$^2$ or more. The number N of the hollow fiber membranes 22 per square centimeter is calculated from the cross-sectional area Sh of the housing 21 and the number M of the hollow fiber membranes 22 accommodated in the housing 21. The product N × D is more preferably 6.5 pieces·cm/cm$^2$ or more, and still more preferably 8 pieces·cm/cm$^2$ or more. The product N × D is preferably 15 pieces·cm/cm$^2$ or less.

As the hollow fiber membrane 22 is denser, the cross-sectional area of the secondary flow passage 24 with respect to the cross-sectional area of the housing 21 is reduced, and thus the flow rate of permeation from the primary flow passage 23 to the secondary flow passage 24 can be suppressed. In a case where N × D ≥ 5 pieces·cm/cm$^2$, the flow rate of permeation can be effectively suppressed, and as a result, the ratio of the flow rate of bypass can be suppressed.

It is preferable that a contact angle of water on the wall surface of the primary flow passage 23 of the hollow fiber membrane 22 is 75° or less. That is, the hollow fiber membrane 22 is preferably hydrophilic. In a case where the contact angle of water is 75° or less, a hydrophobic clogging material is less likely to adhere to the membrane surface, and thus the clogging and the deposition on the wall surface can be suppressed. The contact angle of water is measured by cutting open the hollow fiber membrane to form a flat membrane and dropping 1 μL of a water droplet onto the surface thereof. As the contact angle, a value automatically calculated by a θ/2 method using a device is used. As the device, for example, DropMaster DMs-401 manufactured by Kyowa Interface Science Co., Ltd. is used.

By reversing the direction of the feeding of the culture solution 30 at a preset timing, a flushing effect on the attachment such as the clogging material, adhering to the wall surface of the hollow fiber membrane 22, can be obtained.

In the method for manufacturing a product according to the present disclosure, it is preferable to use a perfusion culture including a step of recovering the product and a step of supplying a fresh culture medium to the tank, in addition to the step of culturing the cells producing the product and the separation step of separating the cells and the product in the culture solution.

The perfusion culture is a culture method in which the fresh culture medium is added and the used culture medium is removed at the same time. According to the perfusion culture, it is possible to achieve a high cell density exceeding 100 × 10$^6$ cells/mL. In addition, the advantage of the perfusion culture is that the culture for producing the target product is maintained for a longer period than a batch culture method or a fed-batch culture.

In the method for manufacturing a product according to the present embodiment, a cell density of the culture solution is preferably 30 × 10$^6$ cells/mL or more. The cell density of the culture solution is more preferably 50 × 10$^6$ cells/mL or more, still more preferably 80 × 10$^6$ cells/mL or more, even more preferably 120 × 10$^6$ cells/mL or more, and even still more preferably 150 × 10$^6$ cells/mL or more. In addition, the cell density of the culture solution is preferably 400 × 10$^6$ cells/mL or less, and more preferably 250 × 10$^6$ cells/mL or less.

In a case where the cell density is high, such as 30 × 10$^6$ cells/mL or more, the membrane clogging is likely to occur, so that the effect of the technology of the present disclosure is high.

The cell density can be determined by measuring the number of cells according to a general method and dividing the number of cells by the amount of the culture solution.

In the method for manufacturing a product according to the present embodiment, a titer of the product is preferably 1.0 g/L or more. The titer of the product is more preferably 1.4 g/L or more, and still more preferably 1.8 g/L or more.

The titer of the product is measured by passing the culture solution through a 0.2 μm syringe filter to remove coarse substances such as the cells, using a metabolic measurement device. As the metabolic measurement device, Cedexbio manufactured by F. Hoffmann-La Roche Ltd. is used.

"Experiment by simple experimental system"

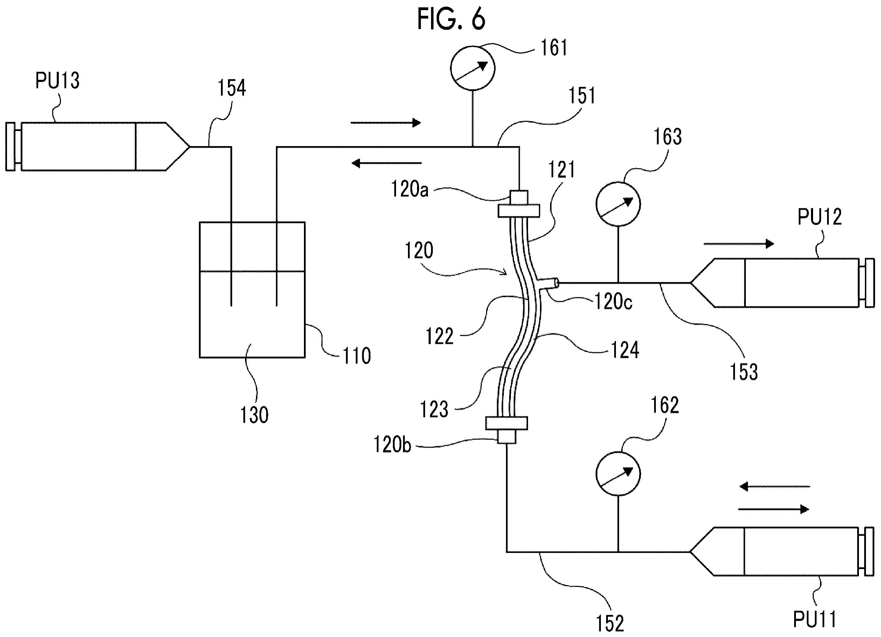

The inventors prepare a simple experimental system, and study a configuration of the hollow fiber membrane filter 20 which can extend the service life of the hollow fiber membrane filter 20 by delaying the membrane clogging of the hollow fiber membrane. FIG. 6 is a schematic view showing a configuration of a simple experimental system 102.

The simple experimental system 102 includes a container 110 which accommodates a filtration target liquid 130 containing the clogging material, a simple filter 120, a first pump PU11, a second pump PU12, a third pump PU13, and flow passages 151 to 154.

The simple filter 120 is prepared from one hollow fiber membrane 122 used in the hollow fiber membrane filter 20 and a polypropylene tube 121 which accommodates the one hollow fiber membrane 122. As shown in FIG. 6, a first opening portion 120a and a second opening portion 120b are provided at both ends of the tube 121. The first opening portion 120a is connected to the flow passage 151 for feeding the filtration target liquid 130 from the container 110 to the simple filter 120. The second opening portion 120b is connected to the flow passage 152 for feeding the filtration target liquid 130 from the simple filter 120 to the pump PU11. The first opening portion 120a and the second opening portion 120b are configured to allow the filtration target liquid 130 to flow into and out of a primary flow passage 123 formed by a hollow portion of the hollow fiber membrane 122. In addition, a discharge port 120c to which the flow passage 153 for discharging a permeated liquid in a secondary flow passage 124 to the outside of the tube 121 is connected is provided on the outer periphery of the tube 121.

The first pump PU11 causes the filtration target liquid 130 to flow from the first opening portion 120a to the simple filter 120 and to be discharged from the second opening portion 120b, and causes the filtration target liquid 130 to flow from the second opening portion 120b to the simple filter 120 and to be discharged from the first opening portion 120a, thereby generating a reciprocating flow of forward feeding and reverse feeding.

The second pump PU12 is a pump which is connected to the flow passage 153 and recovers the permeated liquid in the secondary flow passage 124 through the discharge port 120c.

The third pump PU13 is a pump which is connected to the flow passage 154 and supplies the filtration target liquid 130 to the container 110 through the flow passage 154.

Pressure gauges 161 to 163 are provided in the flow passages 151, 152, and 153, respectively. A first pressure P1, which is a liquid feeding pressure at one end of the primary flow passage 123, is measured by the pressure gauge 161 provided in the flow passage 151. A second pressure P2, which is a liquid feeding pressure at the other end of the primary flow passage 123, is measured by the pressure gauge 162 provided in the flow passage 152. A third pressure P3, which is a liquid feeding pressure of the secondary flow passage 124, is measured by the pressure gauge 163 provided in the flow passage 153.

In the simple experimental system 102, the occurrence of membrane clogging in a case where the length of the hollow fiber membrane 122 is changed is examined. As the filtration target liquid 130, a culture supernatant is used. The culture supernatant is an upper liquid portion in a case where cells are precipitated by centrifuging the culture solution, and contains an antibody and an aggregate of the antibody, which is the clogging material.

The pumps PU11, PU12, and PU13 are operated such that the flow rate of the permeated liquid permeating the hollow fiber membrane 122 is approximately 20 times the flow rate in the actual manufacturing of the product.

For the hollow fiber membranes having a membrane length of 43 cm, 22 cm, and 14 cm, which is the length of the hollow fiber membrane 22, a change in the transmembrane pressure difference [psi] with respect to the amount of the filtrate [$m^3/m^2$] per membrane area is examined. It is noted that 1 psi is 6894.76 Pa.

As the membrane length is longer, the transmembrane pressure difference increases with a smaller amount of the filtrate. Specifically, as the membrane length is longer, the amount of the filtrate per membrane area until the predetermined transmembrane pressure difference (for example, 4.0 psi) is smaller; and as the membrane length is shorter, the amount of the filtrate per membrane area until the transmembrane pressure difference reaches 4.0 psi is larger. That is, it is found that, as the membrane length is shorter, the service life of the hollow fiber membrane filter can be extended for a long period.

Next, the maximum flow rate of bypass for each membrane length is calculated. The maximum flow rate of bypass is a flow rate of bypass at an end part of the hollow fiber membrane in the longitudinal direction. The flow rate of bypass per membrane area per hour is calculated under a condition that the amount of the filtrate per membrane area is constant. As a result, it is found that, as the membrane length is shorter, the flow rate of bypass can be reduced. Specifically, in a case where the membrane lengths of 43 cm, 22 cm, and 14 cm are compared, in a case of the membrane length of 22 cm, the maximum flow rate of bypass is half or less as compared with a case of the membrane length of 43 cm, and in a case of the membrane length of 14 cm, the maximum flow rate of bypass is 1/3 or less.

From the results of the membrane clogging in the simple experimental system and the calculation results of the maximum flow rate of bypass, it is clarified that, the flow rate of bypass is larger, the membrane clogging occurs earlier, and the membrane clogging can be delayed by suppressing the bypass flow.

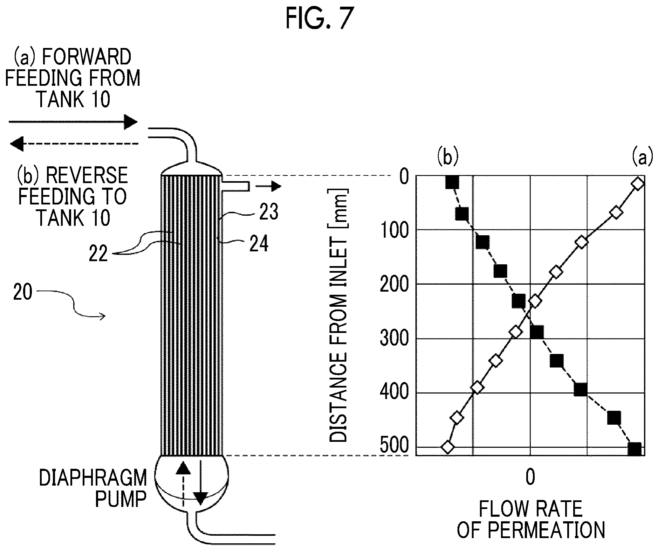

FIG. 7 shows changes in the flow rate of permeation in the length direction of the hollow fiber membrane 22 in a case (a) where the diaphragm pump (corresponding to the first pump PU1 in FIG. 1) connected to the hollow fiber membrane filter 20 is operated to feed the culture solution forward from the tank 10 to the hollow fiber membrane filter 20 and in a case (b) where the culture solution is fed backward from the hollow fiber membrane filter 20 to the tank 10. FIG. 7 is a calculation result based on a mathematical model. In the mathematical model, the length L of the hollow fiber membrane 22 is set to 520 mm, and the cell density of the culture solution is set to $120 \times 10^6$ cells/mL.

In FIG. 7, the position of the hollow fiber membrane 22 in the length direction is indicated by a distance (distance from the inlet in FIG. 7) from the reference position, where the reference position is one end of the hollow fiber membrane 22 of the hollow fiber membrane filter 20 on the tank 10 side. As described above, the flow rate of permeation is an amount of the permeated liquid permeating from the primary flow passage 23 to the secondary flow passage 24 of the hollow fiber membrane 22 per unit area per unit time.

As shown in FIG. 7, the flow rate of permeation is maximized at one end of the hollow fiber membrane 22 on the supply side. There is a correlation between the flow rate of permeation and the amount of clogging materials deposited on the membrane surface of the hollow fiber membrane 22, and as the flow rate of permeation is larger, a thickness of the deposition layer is larger.

From the results of the above-described simple experiment, the mathematical model, and the like, the mechanism of the occurrence of the bypass flow and the membrane clogging of the hollow fiber membrane filter is presumed as follows.

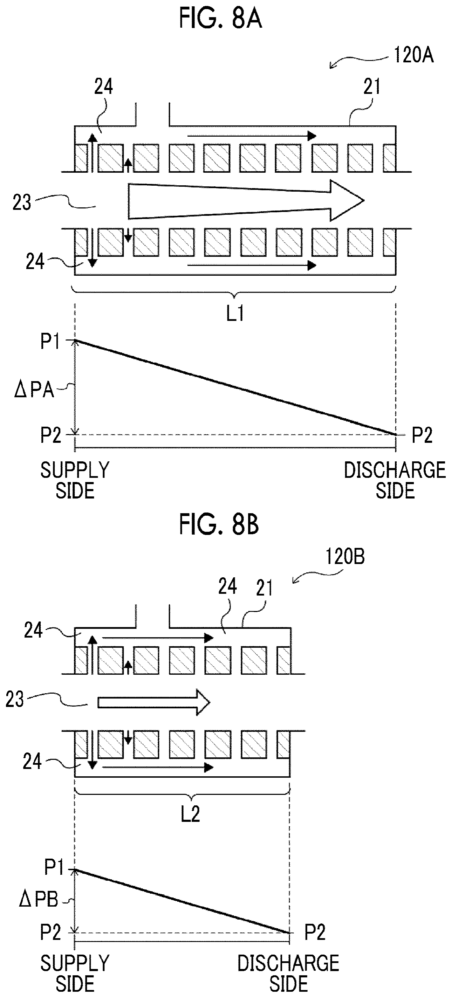

FIG. 8A is a view for describing a mechanism of occurrence of a bypass flow in a hollow fiber membrane filter 120A having a hollow fiber membrane with a relatively long membrane length L1 and FIG. 8B is a view for describing a mechanism of occurrence of a bypass flow in a hollow fiber membrane filter 120B having a hollow fiber membrane with a relatively short membrane length L2. In a case where the flow rate of filtration per unit membrane area is the same, the pressure difference $\Delta P$ at both ends of the primary flow passage 23 increases as the membrane length increases. That is, in a case where the flow rate of filtration per unit membrane area is the same in the hollow fiber membrane filters 120A and 120B, as shown in FIG. 8A, the pressure difference $\Delta PA$ at both ends of the primary flow passage 23 of the hollow fiber membrane filter 120A is larger than the pressure difference $\Delta PB$ at both ends of the primary flow passage 23 of the hollow fiber membrane filter 120B. Therefore, in the primary flow passage 23 of the hollow fiber membrane filter 120A, the flow toward the wall surface on the supply side is likely to occur, the flow rate of bypass is likely to increase, and the membrane clogging is likely to progress. On the other hand, in the primary flow passage 23 of the hollow fiber membrane filter 120B in FIG. 8B, since the pressure difference $\Delta P$ at both ends is smaller than that of the hollow fiber membrane filter 120A, the flow toward the wall surface on the supply side is relatively small. Accordingly, the flow rate of bypass is also relatively small, and as a result, the progress speed of the membrane clogging can be reduced.

In a case where the tank capacity (that is, the amount of the culture solution) is large, the amount of the recovered filtrate also increases proportionally. In a case where a relationship between the membrane area of the hollow fiber membrane 22 to be used and the amount of the culture solution to be fed is scaled up with a constant membrane area/amount of culture solution, the amount of the permeated liquid per unit membrane area does not change, and thus it is necessary to increase the membrane area itself. In a case where the hollow fiber membrane 22 is scaled up to increase the membrane area, the membrane area is generally increased by increasing the length of the hollow fiber membrane. However, according to the above-described study, it is clarified that, in a case where the hollow fiber membrane is simply lengthened, as the hollow fiber membrane is longer, the differential pressure at both ends is larger and the bypass flow increases, so that the service life is shortened. Therefore, in the method for manufacturing a product in which the culture is performed using the tank 10 having a capacity of 100 L or more, it is effective to reduce the pressure difference $\Delta P$ at both ends of the primary flow passage 23 of the hollow fiber membrane 22 during the feeding of the culture solution, specifically, to extend the service life for a long period of time by setting $\Delta P/\mu$ to $4.9 \times 10^6$ [1/s] or less.

The manufactured product can be used, for example, in a biopharmaceutical, regenerative medicine, and the like.

In the above-described embodiments, the example has been described in which the hollow fiber membrane filter 20 is used for separating the cells and the product in the culture solution, but the hollow fiber membrane filter 20 can also be applied to a treatment of separating solid particles, not limited to the separation of the cells and the product. The maximum length of the solid particles is 1 $\mu$m or more, preferably 2 $\mu$m or more, and more preferably 5 $\mu$m or more. The maximum length thereof is preferably 100 $\mu$m or less, more preferably 50 $\mu$m or less, and still more preferably 10 $\mu$m or less.

In addition, in the above-described embodiments, as a hardware structure of the processor, various processors shown below can be used. Various processors include a programmable logic device (PLD) that is capable of changing a circuit configuration after manufacturing, such as a field-programmable gate array (FPGA), and a dedicated electric circuit that is a processor having a circuit configuration dedicatedly designed for executing specific process, such as an application specific integrated circuit (ASIC), in addition to a CPU that is a general-purpose processor configured to execute software (program) to function as various processing units.

In addition, the above-described process may be executed by one of various processors or may be executed by a combination of two or more processors (for example, a combination of a plurality of FPGAs or a CPU and an FPGA) of the same type or different types. The plurality of processing units may be configured of one processor. As an example in which a plurality of processing units are composed of one processor, there is a form in which a processor that realizes all functions of a system including a plurality of processing units into one integrated circuit (IC) chip is used, such as system on chip (SOC).

Furthermore, the hardware structure of the processors is, more specifically, an electric circuit (circuitry) in which circuit elements such as semiconductor elements are combined.

In addition, the technology of the present disclosure is applied to not only the operation program of the analysis apparatus but also a non-transitory computer readable storage medium (a USB memory, a digital versatile disc (DVD)-read only memory (ROM), or the like) storing the operation program of the analysis apparatus.

Examples

Hereinafter, the embodiments of the present disclosure will be described in more detail by Examples.

A product was manufactured by the methods for manufacturing a product of Comparative Example 1 and Comparative Example 2, using an apparatus having a configuration substantially equivalent to that of the cell culture apparatus shown in FIG. 1. In addition, simulations were performed for the methods for manufacturing a product of Examples 1 to 8. The target continuous operation period was 34 days.

"Comparative Example 1"

First, 44 L of a culture medium was put into the tank and held at 37°C, and air and $CO_2$ were aerated from an upper surface of the tank 10 and left for 1 day. Next, cells were seeded such that a cell density in the tank was $5.0 \times 10^5$ cells/mL and an amount of liquid in the tank was 45 L. From 2 days after the seeding, the culture solution was continuously extracted by the pump, the cells and the product were separated (separation step), and the fresh culture medium was supplied (step of supplying the fresh culture medium) at a rate of 27 L/day. In addition, from 5 days after the seeding, the culture solution was continuously extracted by the pump, the cells and the product were separated (separation step), and the fresh culture medium was supplied (step of supplying the fresh culture medium) at a rate of 54 L/day. In the separation step, the culture solution which did not permeate through the secondary flow passage was returned to the tank, and the permeated liquid which permeated through the secondary flow passage was recovered to recover the antibody as the product (recovery step). The cultured cells were CHO cells which produced an antibody. As the culture medium, a culture medium adjusted with reference to the exchange culture medium in Table 2 of JP2000-517188A was used.

The separation step, the step of supplying the fresh culture medium, and the recovery step were continued for 60 days or until a predetermined membrane clogging state, with the day on which the separation step was started being defined as the first day.

As the hollow fiber membrane filter, a hollow fiber membrane filter in which a housing having an inner diameter Dh = 53.2 mm was provided and M = 830 hollow fiber membranes were accommodated in the housing was used. The hollow fiber membrane had a membrane length L = 295 mm, an inner diameter d = 1 mm, an outer diameter D = 1.26 mm, and a membrane thickness (D - d)/2 = 0.13 mm. In this case, L/d = 295 and D/d = 1.26. In addition, in a cross-sectional area perpendicular to a length direction of the hollow fiber membrane filter, a housing cross-sectional area Sh = $\pi Dh^2$ = 2222.9 $mm^2$, a primary flow passage cross-sectional area S1 = M $\times \pi(d/2)^2$ = 651.9 $mm^2$, a secondary flow passage cross-sectional area A = Sh - M $\times \pi(D/2)^2$ = 1187.9 $mm^2$, a ratio of the primary flow passage cross-sectional area S1 to the housing cross-sectional area Sh is S1/Sh = 0.29, and the number of the hollow fiber membranes per 1 $cm^2$, N = M/Sh = 37.34. In addition, a hydraulic radius reff = $\pi(Dh^2 - M \cdot D^2)/2\pi(Dh + M \cdot D)$ = 0.688. A pressure loss coefficient k of the hollow fiber membrane was $5.548 \times 10^{14}$/m², a surface roughness Sa of the membrane surface was 7.9 mm, and a contact angle was 70°.

A viscosity $\mu$ of the culture solution to be fed was 2.0 mPa·s, and a pressure difference $\Delta P$ at both ends of the primary flow passage 23 during the feeding was set to 1.44 psi. That is, $\Delta P/\mu$ was set to 4.96.

In this case, a hollow fiber membrane resistance r = $\mu k \cdot \ln(D/d)/2\pi L$ = 1203.9 psi/mL/min, a flow resistance R of the secondary flow passage = $8\mu LM/\pi A(reff)^2$ = 19.3 psi/mL/min, and $\Delta P/2(r + R)$ = 0.00059 mL/min.

"Comparative Example 2"

As the hollow fiber membrane filter, a hollow fiber membrane filter in which a housing having an inner diameter Dh = 16.3 mm was provided and M = 75 hollow fiber membranes were accommodated in the housing was used. The hollow fiber membrane had a membrane length L = 558.5 mm, an inner diameter d = 1 mm, an outer diameter D = 1.26 mm, and a membrane thickness (D - d)/2 = 0.13 mm. In this case, L/D = 558.5 and D/d = 1.26. In addition, in a cross-sectional area perpendicular to a length direction of the hollow fiber membrane filter, a housing cross-sectional area Sh = 208.7 $mm^2$, a primary flow passage cross-sectional area S1 = 158.9 $mm^2$, a secondary flow passage cross-sectional area A = 115.2 $mm^2$, a ratio of the primary flow passage cross-sectional area S1 to the housing cross-sectional area Sh was S1/Sh = 0.28, and the number of the hollow fiber membranes per 1 $cm^2$ was N = 35.94. In addition, a hydraulic radius reff was 0.662. Since the material and the membrane thickness of the hollow fiber membrane were the same as those in Comparative Example 1, the pressure loss coefficient k, the surface roughness Sa of the membrane surface, and the contact angle were the same as those in Comparative Example 1. The culture solution to be fed was also the same as that in Comparative Example 1.

A pressure difference $\Delta P$ at both ends of the primary flow passage during the feeding was set to 3.51 psi. That is, $\Delta P/\mu$ was set to 12.10.

In this case, a hollow fiber membrane resistance r = 635.9 psi/mL/min, a flow resistance R of the secondary flow passage = 36.8 psi/mL/min, and $\Delta P/2(r + R)$ = 0.00261 mL/min.

<Evaluation of membrane clogging>

For Comparative Examples 1 and 2, the degree of clogging of the hollow fiber membrane filter was measured at the same time every day from the day on which the separation step was started, and the number of days until TMP = 1.5 psi and the number of days until $\Delta$Pin = 4 psi were examined. Here, TMP is a transmembrane pressure difference between the primary flow passage 23 and the secondary flow passage 24, and increased as the clogging of the pores of the porous membrane increased in the membrane clogging, and thus was used as an indicator for evaluating the degree of clogging of the pores (clogging of the pores) in the membrane clogging. In addition, $\Delta$Pin is an amount of increase in the liquid feeding pressure (second pressure P2) at the end part of the primary flow passage on the first pump PU1 side from the initial value. ΔPin increased as the primary flow passage 23 was clogged, and thus was used as an indicator for evaluating the deposition state on the membrane surface in the membrane clogging.

The first pressure P1, the second pressure P2, and the third pressure P3 were measured, and TMP = (P1_avg + P2_avg)/2 - P3_avg. Here, P1_avg = (P1_max + P1_min)/2, and P1_max and P1_min indicate maximum and minimum values of the first pressure P1 in the culture solution reciprocating five times in the hollow fiber membrane, respectively. The same applies to P2_avg and P3_avg.

ΔPin = (P2max - P2min) - (P2i_max - P2i_min) Here, P2i is an initial value of P2 (second pressure on the day on which the separation step was started).

"Examples 1 to 8"

In Examples 1 to 8, the configuration of the hollow fiber membrane filter was as shown in Table 1, and the membrane clogging was evaluated.

As shown in Table 1, for example, in Example 1, a hollow fiber membrane filter including a housing having an inner diameter Dh = 53.2 mm and accommodating M = 1,000 hollow fiber membranes in the housing was used as the hollow fiber membrane filter. The hollow fiber membrane had a membrane length L = 220 mm, an inner diameter d = 1 mm, an outer diameter D = 1.26 mm, and a membrane thickness (D - d)/2 = 0.13 mm. In this case, L/D = 220 and D/d = 1.26. In addition, in a cross-sectional area perpendicular to a length direction of the hollow fiber membrane filter, a housing cross-sectional area $Sh = 2222.9 \text{ mm}^2$, a primary flow passage cross-sectional area $S1 = 785.4 \text{ mm}^2$, a secondary flow passage cross-sectional area $A = 976 \text{ mm}^2$, a ratio of the primary flow passage cross-sectional area S1 to the housing cross-sectional area Sh was S1/Sh = 0.353, and the number of the hollow fiber membranes per $1 \text{ cm}^2$ was N = 44.99. In addition, a hydraulic radius reff was 0.473. The material and the membrane thickness of the hollow fiber membrane were the same as those of Comparative Example 1. In addition, the culture solution to be fed was also the same as that in Comparative Example 1.

A pressure difference ΔP at both ends of the primary flow passage during the feeding was set to 0.8 psi. That is, ΔP/μ was set to 2.76.

In this case, a hollow fiber membrane resistance r = 1614.4 psi/mL/min, a flow resistance R of the secondary flow passage = 44.6 psi/mL/min, and ΔP/2(r + R) = 0.00024 mL/min.

The effects of Examples 1 to 8 were also evaluated in terms of number of days until TMP = 1.5 psi and the number of days until ΔPin = 4 psi.

Table 1 summarizes the configuration of the hollow fiber membrane filter, the liquid feeding condition, and the evaluation result of the membrane clogging as the effect for Comparative Examples 1 and 2 and Examples 1 to 8. In Table 1, "No occurrence" in the number of days until ΔPin = 4 psi indicates that ΔPin did not reach 4 psi on the 60th day.

[Table 1]

| | | Unit | Example 1 | Example 2 | Example 3 | Example 4 | Example 5 | Example 6 | Example 7 | Example 8 | Comparative Example 1 | Comparative Example 2 |
|---|---|---|---|---|---|---|---|---|---|---|---|---|---|
| Configuration of hollow fiber membrane filter | Membrane length L of hollow fiber membrane | mm | 220 | 140 | 220 | 220 | 220 | 220 | 220 | 220 | 295 | 558.5 |
| | Inner diameter d of hollow fiber membrane | mm | 1 | 1 | 2 | 4 | 1 | 1 | 1 | 1 | 1 | 1 |
| | Outer diameter D of hollow fiber membrane | mm | 1.26 | 1.26 | 2.52 | 5.04 | 1.4 | 1.6 | 1.26 | 1.26 | 1.26 | 1.26 |
| | Membrane thickness of hollow fiber membrane | mm | 0.13 | 0.13 | 0.26 | 0.52 | 0.2 | 0.3 | 0.13 | 0.13 | 0.13 | 0.13 |
| | Number M of hollow fiber membranes | pieces | 1000 | 1000 | 512 | 238 | 1000 | 1000 | 1200 | 1500 | 830 | 75 |
| | Inner diameter Dh of housing | mm | 53.2 | 53.2 | 67.3 | 85.2 | 57.8 | 59 | 53.2 | 60 | 53.2 | 16.3 |
| | L/d | - | 220 | 140 | 110 | 55 | 220 | 220 | 220 | 220 | 295 | 558.5 |
| | Primary flow passage cross-sectional area S1 | mm$^2$ | 785.4 | 785.4 | 1608.5 | 2990.8 | 785.4 | 785.4 | 942.5 | 1178.1 | 651.9 | 58.9 |
| | Secondary flow passage cross-sectional area A | mm$^2$ | 976.0 | 976.0 | 1003.6 | 953.0 | 1084.5 | 723.4 | 726.6 | 957.1 | 1187.9 | 115.2 |
| | Housing cross-sectional area Sh | mm$^2$ | 2222.9 | 2222.9 | 3557.3 | 5701.2 | 2623.9 | 2734.0 | 2222.9 | 2827.4 | 2222.9 | 208.7 |
| | S1/Sh | - | 0.353 | 0.353 | 0.452 | 0.525 | 0.299 | 0.287 | 0.424 | 0.417 | 0.29 | 0.28 |
| | N | pieces/cm$^2$ | 44.99 | 44.99 | 14.39 | 4.17 | 38.11 | 36.58 | 53.98 | 53.05 | 37.34 | 35.94 |
| | D/d | - | 1.26 | 1.26 | 1.26 | 1.26 | 1.4 | 1.6 | 1.26 | 1.26 | 1.26 | 1.26 |
| | Hydraulic radius reff | mm | 0.473 | 0.473 | 0.471 | 0.472 | 0.474 | 0.278 | 0.296 | 0.312 | 0.688 | 0.662 |
| | Pressure loss coefficient k of hollow fiber membrane | $\times 10^{14}/m^2$ | 5.548 | 5.548 | 5.548 | 5.548 | 5.548 | 5.548 | 5.548 | 5.548 | 5.548 | 5.548 |
| | Surface roughness Sa | μm | 7.9 | 7.9 | 7.9 | 7.9 | 7.9 | 7.9 | 7.9 | 7.9 | 7.9 | 7.9 |
| | Contact angle | ° | 70 | 70 | 70 | 70 | 70 | 70 | 70 | 70 | 70 | 70 |
| Liquid feeding condition | Viscosity μ of culture solution | mPa·s | 2 | 2 | 2 | 2 | 2 | 2 | 2 | 2 | 2 | 2 |
| | Pressure difference ΔP at both ends of primary flow passage | psi | 0.8 | 0.6 | 0.4 | 0.2 | 0.8 | 0.8 | 0.7 | 0.6 | 1.44 | 3.51 |
| | ΔP/μ | $\times 10^6$ [1/s] | 2.76 | 2.07 | 1.38 | 0.69 | 2.76 | 2.76 | 2.41 | 2.07 | 4.96 | 12.10 |
| | Membrane resistance r of hollow fiber membrane | psi/mL/min | 1614.4 | 2536.9 | 1614.4 | 1614.4 | 2350.3 | 3283.1 | 1614.4 | 1614.4 | 1203.9 | 635.9 |
| | Flow passage resistance R of secondary flow passage | psi/mL/min | 44.6 | 28.4 | 22.5 | 10.9 | 40.1 | 174.9 | 184.4 | 156.5 | 19.3 | 36.8 |
| | ΔP/2(r + R) | mL/min | 0.00024 | 0.00012 | 0.00012 | 0.00006 | 0.00017 | 0.00012 | 0.00019 | 0.00017 | 0.00059 | 0.00261 |
| Effect | Number of days until TMP = 1.5 psi | days | 40 | 50 | 55 | 60 | 42 | 42 | 45 | 45 | 27 | 22 |
| | Number of days until ΔPin = 4 psi | days | No occurrence | No occurrence | No occurrence | No occurrence | No occurrence | No occurrence | No occurrence | No occurrence | No occurrence | 28 |

As shown in Table 1, in Examples 1 to 8 in which ΔP/μ during the feeding of the culture solution satisfied $4.9 \times 10^6$ [1/s] or less, ΔPin indicating the degree of clogging did not reach 4 psi even after 60 days, and the number of days until TMP reached 1.5 psi also exceeded the target of 34 days. On the other hand, in Comparative Examples 1 and 2 in which ΔP/μ did not satisfy $4.9 \times 10^6$ [1/s] or less, the number of days until TMP reached 1.5 psi was less than 34 days. In Comparative Example 1, ΔPin did not exceed 4 psi even after 60 days, but it is presumed that the efficiency was significantly reduced at the point of 60 days.

From the comparison of Examples 1 to 4, it was found that, as L/d was smaller, the period until the membrane clogging (service life) was longer. From the results of Examples 1 to 4, it was found that L/d is preferably 220 or less, more preferably 140 or less, and still more preferably 110 or less.

All of Examples 1 to 8 satisfied S1/Sh ≥ 0.28. From the comparison of Examples 1, 3, and 4, it was found that, as S1/Sh was larger, the service life could be made longer.

From the comparison of Examples 1, 5, and 6, it was found that, as D/d was larger, the service life could be made longer.

From the comparison of Examples 1, 7, and 8, it was found that, as the hydraulic radius reff was smaller as 0.55 or less, the service life could be made longer.

The contents described and shown above are detailed descriptions of portions according to the technology of the present disclosure and are merely examples of the technology of the present disclosure. For example, the above description of the configurations, functions, operations, and effects is the description of examples of the configurations, functions, operations, and effects of portions related to the technology of the present disclosure. Therefore, unnecessary portions may be deleted or new elements may be added or replaced in the above descriptions and illustrations without departing from the gist of the technology of the present disclosure. In addition, in order to avoid complication and facilitate understanding of portions according to the technology of the present disclosure, description related to common technical knowledge or the like that does not need to be particularly described for enabling implementation of the technology of the present disclosure is omitted in the contents described and shown above.

The disclosure of Japanese Patent Application No. 2023-095894 filed on June 9, 2023 is incorporated in the present specification by reference.

All cited documents, patent applications, and technical standards described in the specification are incorporated by reference in the specification to the same extent as in a case where each individual cited document, patent application, or technical standard is specifically and individually indicated to be incorporated by reference.

In regard to the above-described embodiments, following supplementary notes are further described.

<Supplementary note 1>

A method for manufacturing a product obtained by culturing cells, the method including:

a culture step of culturing the cells in a culture solution accommodated in a tank having a capacity of 100 L or more; and
a separation step of using a hollow fiber membrane filter which has a hollow fiber membrane having a primary flow passage extending in a longitudinal direction inside the hollow fiber membrane and has a housing accommodating a plurality of the hollow fiber membranes, the hollow fiber membrane filter having a secondary flow passage in a space of the housing outside each of the hollow fiber membranes, to feed the culture solution supplied from the tank to the primary flow passage, and allowing a permeated liquid containing the product to permeate through the secondary flow passage to separate the cells and the product in the culture solution from each other,
in which, in the separation step, in a case where a difference in pressure between both ends of the primary flow passage on a supply side and a discharge side during the feeding of the culture solution is denoted as $\Delta P$ [Pa], and a viscosity of the culture solution is denoted as $\mu$ [Pa·s], $\Delta P/\mu$ during the feeding of the culture solution is $4.9 \times 10^6$ [1/s] or less.

<Supplementary note 2>

The method for manufacturing a product according to the supplementary note 1,
in which a relationship between a length L of the hollow fiber membrane and an inner diameter d of the hollow fiber membrane satisfies $L/d \leq 290$.

<Supplementary note 3>

The method for manufacturing a product according to the supplementary note 1 or 2,
in which a relationship between a total cross-sectional area S1 of the primary flow passage in a cross section perpendicular to a length direction of the hollow fiber membrane filter and a cross-sectional area Sh of the housing satisfies $S1/Sh \geq 0.28$.

<Supplementary note 4>

The method for manufacturing a product according to any one of the supplementary notes 1 to 3,
in which a membrane resistance r of the hollow fiber membrane is 1,250 psi/mL/min or more.

<Supplementary note 5>

The method for manufacturing a product according to any one of the supplementary notes 1 to 4,
in which a pressure loss coefficient k between the primary flow passage and the secondary flow passage of the hollow fiber membrane is $5 \times 10^{13}/m^2$ or more.

<Supplementary note 6>

The method for manufacturing a product according to any one of the supplementary notes 1 to 5,
in which a relationship between an outer diameter D and an inner diameter d of the hollow fiber membrane satisfies $D/d \geq 1.1$.

<Supplementary note 7>

The method for manufacturing a product according to any one of the supplementary notes 1 to 6,

in which a surface roughness Sa of a wall surface of the primary flow passage of the hollow fiber membrane is 8 $\mu$m or less.

<Supplementary note 8>

The method for manufacturing a product according to any one of the supplementary notes 1 to 7, in which, in the separation step, a direction of the feeding is reversed at a preset timing.

<Supplementary note 9>

The method for manufacturing a product according to any one of the supplementary notes 1 to 8, in which the product is an antibody.

<Supplementary note 10>

The method for manufacturing a product according to any one of the supplementary notes 1 to 9, in which the method is a perfusion culture including a step of recovering the product and a supply step of supplying a culture medium to the tank.

<Supplementary note 11>

The method for manufacturing a product according to any one of the supplementary notes 1 to 10, in which a cell density of the culture solution is $100 \times 10^6$ cells/mL or more.

<Supplementary note 12>

The method for manufacturing a product according to any one of the supplementary notes 1 to 11, in which a titer of the product is 1.0 g/L or more.

<Supplementary note 13>

A hollow fiber membrane filter used for a method for manufacturing a product obtained by culturing cells in a culture solution accommodated in a tank having a capacity of 100 L or more, the hollow fiber membrane filter including:

a hollow fiber membrane having a primary flow passage extending in a longitudinal direction inside the hollow fiber membrane; and
a housing accommodating a plurality of the hollow fiber membranes,
in which a length L of the hollow fiber membrane and an inner diameter d of the hollow fiber membrane each satisfy L $\leq$ 290 mm and d $\geq$ 1 mm, and
a relationship between the length L and the inner diameter d satisfies L/d $\leq$ 290.

<Supplementary note 14>

The hollow fiber membrane filter according to the supplementary note 13, in which a product N $\times$ D of a number N of the hollow fiber membranes per square centimeter in a cross section perpendicular to a length direction of the housing and an outer diameter D of the hollow fiber membrane is 5 pieces·cm/cm$^2$ or more.

<Supplementary note 15>

The hollow fiber membrane filter according to the supplementary note 13 or 14, in which a relationship between a total cross-sectional area S1 of the primary flow passage in a cross section perpendicular to a length direction of the hollow fiber membrane filter and a cross-sectional area Sh of the housing satisfies S1/Sh $\geq$ 0.28.

<Supplementary note 16>

The hollow fiber membrane filter according to any one of the supplementary notes 13 to 15, in which a pressure loss coefficient k of the hollow fiber membrane is $5 \times 10^{13}$/m$^2$ or more.

<Supplementary note 17>

The hollow fiber membrane filter according to any one of the supplementary notes 13 to 16, in which a relationship between an outer diameter D and the inner diameter d of the hollow fiber membrane satisfies D/d ≥ 1.1.

<Supplementary note 18>

The hollow fiber membrane filter according to any one of the supplementary notes 13 to 17, in which a hydraulic radius reff represented by the following expression using a number M of the hollow fiber membranes accommodated in the housing, an inner diameter $D_h$ of the housing, and an outer diameter D of the hollow fiber membrane is 0.55 or less,

$$\text{reff} = \pi(D_h^2 - M{\cdot}D^2)/2\pi(D_h + M{\cdot}D).$$

<Supplementary note 19>

The hollow fiber membrane filter according to any one of the supplementary notes 13 to 18, in which a contact angle of water on a wall surface of the primary flow passage of the hollow fiber membrane is 75° or less.

<Supplementary note 20>

An apparatus for manufacturing a product used for a method for manufacturing a product obtained by culturing cells in a culture solution, the apparatus including:

a tank having a capacity of 100 L or more, the tank accommodating the culture solution;
a hollow fiber membrane filter which has a hollow fiber membrane having a primary flow passage extending in a longitudinal direction inside the hollow fiber membrane and a housing accommodating a plurality of the hollow fiber membranes, in which a length L of the hollow fiber membrane and an inner diameter d of the hollow fiber membrane each satisfy L ≤ 290 mm and d ≥ 1 mm, and a relationship between the length L and the inner diameter d satisfies L/d ≤ 290;
a first pump which feeds the culture solution to the primary flow passage of the hollow fiber membrane filter; and
a second pump which feeds, to a product recovery part, a permeated liquid passing through a secondary flow passage of the hollow fiber membrane filter in a case where the culture solution is fed along the primary flow passage of the hollow fiber membrane filter. Explanation of References

10: tank
11: stirring blade
20: hollow fiber membrane filter
20a: first opening portion
20b: second opening portion
20c: discharge port
21: housing
22: hollow fiber membrane
22a: porous membrane
22h: pore
23: primary flow passage
24: secondary flow passage
30: culture solution
31: culture medium
32: cell
33: product (antibody)
35: permeated liquid
36: clogging material
40: product recovery part
51 to 54: flow passage
61 to 63: pressure gauge

70: processor
100: cell culture apparatus
102: simple experimental system
110: container
120: simple filter
120A: hollow fiber membrane filter
120B: hollow fiber membrane filter
120a: first opening portion
120b: second opening portion
120c: discharge port
121: tube
122: hollow fiber membrane
123: primary flow passage
124: secondary flow passage
130: filtration target liquid
151 to 154: flow passage
161 to 163: pressure gauge
d: inner diameter of hollow fiber membrane
D: outer diameter of hollow fiber membrane
L: length of hollow fiber membrane (membrane length)
PU1, PU11: first pump
PU2, PU12: second pump
PU3, PU13: third pump

**Claims**

1. A method for manufacturing a product obtained by culturing cells, the method comprising:

   a culture step of culturing the cells in a culture solution accommodated in a tank having a capacity of 100 L or more; and
   a separation step of using a hollow fiber membrane filter which has a hollow fiber membrane having a primary flow passage extending in a longitudinal direction inside the hollow fiber membrane and has a housing accommodating a plurality of the hollow fiber membranes, the hollow fiber membrane filter having a secondary flow passage in a space of the housing outside each of the hollow fiber membranes, to feed the culture solution supplied from the tank to the primary flow passage, and allowing a permeated liquid containing the product to permeate through the secondary flow passage to separate the cells and the product in the culture solution from each other,
   wherein, in the separation step, in a case where a difference in pressure between both ends of the primary flow passage on a supply side and a discharge side during the feeding of the culture solution is denoted as $\Delta P$ [Pa], and a viscosity of the culture solution is denoted as $\mu$ [Pa·s], $\Delta P/\mu$ during the feeding of the culture solution is $4.9 \times 10^6$ [1/s] or less.

2. The method for manufacturing a product according to claim 1,
   wherein a relationship between a length L of the hollow fiber membrane and an inner diameter d of the hollow fiber membrane satisfies $L/d \leq 290$.

3. The method for manufacturing a product according to claim 1,
   wherein a relationship between a total cross-sectional area S1 of the primary flow passage in a cross section perpendicular to a length direction of the hollow fiber membrane filter and a cross-sectional area Sh of the housing satisfies $S1/Sh \geq 0.28$.

4. The method for manufacturing a product according to claim 1,
   wherein a membrane resistance r of the hollow fiber membrane is 1,250 psi/mL/min or more.

5. The method for manufacturing a product according to claim 1,
   wherein a pressure loss coefficient k between the primary flow passage and the secondary flow passage of the hollow fiber membrane is $5 \times 10^{13}/m^2$ or more.

**6.** The method for manufacturing a product according to claim 1,
wherein a relationship between an outer diameter D and an inner diameter d of the hollow fiber membrane satisfies D/d ≥ 1.1.

**7.** The method for manufacturing a product according to claim 1,
wherein a surface roughness Sa of a wall surface of the primary flow passage of the hollow fiber membrane is 8 μm or less.

**8.** The method for manufacturing a product according to any one of claims 1 to 7,
wherein, in the separation step, a direction of the feeding is reversed at a preset timing.

**9.** The method for manufacturing a product according to any one of claims 1 to 7,
wherein the product is an antibody.

**10.** The method for manufacturing a product according to any one of claims 1 to 7,
wherein the method is a perfusion culture including a step of recovering the product and a supply step of supplying a culture medium to the tank.

**11.** The method for manufacturing a product according to any one of claims 1 to 7,
wherein a cell density of the culture solution is $30 \times 10^6$ cells/mL or more.

**12.** The method for manufacturing a product according to any one of claims 1 to 7,
wherein a titer of the product is 1.0 g/L or more.

**13.** A hollow fiber membrane filter used for a method for manufacturing a product obtained by culturing cells in a culture solution accommodated in a tank having a capacity of 100 L or more, the hollow fiber membrane filter comprising:

a hollow fiber membrane having a primary flow passage extending in a longitudinal direction inside the hollow fiber membrane; and
a housing accommodating a plurality of the hollow fiber membranes,
wherein a length L of the hollow fiber membrane and an inner diameter d of the hollow fiber membrane each satisfy L ≤ 290 mm and d ≥ 1 mm, and
a relationship between the length L and the inner diameter d satisfies L/d ≤ 290.

**14.** The hollow fiber membrane filter according to claim 13,
wherein a product N × D of a number N of the hollow fiber membranes per square centimeter in a cross section perpendicular to a length direction of the housing and an outer diameter D of the hollow fiber membrane is 5 pieces·cm/cm$^2$ or more.

**15.** The hollow fiber membrane filter according to claim 13,
wherein a relationship between a total cross-sectional area S1 of the primary flow passage in a cross section perpendicular to a length direction of the hollow fiber membrane filter and a cross-sectional area Sh of the housing satisfies S1/Sh ≥ 0.28.

**16.** The hollow fiber membrane filter according to claim 13,
wherein a pressure loss coefficient k of the hollow fiber membrane is $5 \times 10^{13}$/m$^2$ or more.

**17.** The hollow fiber membrane filter according to claim 13,
wherein a relationship between an outer diameter D and the inner diameter d of the hollow fiber membrane satisfies D/d ≥ 1.1.

**18.** The hollow fiber membrane filter according to claim 13,
wherein a hydraulic radius reff represented by the following expression using a number M of the hollow fiber membranes accommodated in the housing, an inner diameter $D_h$ of the housing, and an outer diameter D of the hollow fiber membrane is 0.55 or less,

$$\mathrm{reff} = \pi(D_h{}^2 - M \cdot D^2)/2\pi(D_h + M \cdot D).$$

19. The hollow fiber membrane filter according to claim 13,
wherein a contact angle of water on a wall surface of the primary flow passage of the hollow fiber membrane is 75° or less.

20. An apparatus for manufacturing a product used for a method for manufacturing a product obtained by culturing cells in a culture solution, the apparatus comprising:

a tank having a capacity of 100 L or more, the tank accommodating the culture solution;
a hollow fiber membrane filter which has a hollow fiber membrane having a primary flow passage extending in a longitudinal direction inside the hollow fiber membrane and a housing accommodating a plurality of the hollow fiber membranes, in which a length L of the hollow fiber membrane and an inner diameter d of the hollow fiber membrane each satisfy $L \leq 290$ mm and $d \geq 1$ mm, and a relationship between the length L and the inner diameter d satisfies $L/d \leq 290$;
a first pump which feeds the culture solution to the primary flow passage of the hollow fiber membrane filter; and
a second pump which feeds, to a product recovery part, a permeated liquid passing through a secondary flow passage of the hollow fiber membrane filter in a case where the culture solution is fed along the primary flow passage of the hollow fiber membrane filter.

FIG. 1

# FIG. 2

FIG. 3

FIG. 4

EP 4 711 467 A1

# FIG. 5

EP 4 711 467 A1

FIG. 6

EP 4 711 467 A1

# FIG. 7

(a) FORWARD
FEEDING FROM
TANK 10

(b) REVERSE
FEEDING TO
TANK 10

22  23  24

20

DIAPHRAGM
PUMP

(b)                    (a)

DISTANCE FROM INLET [mm]

0
100
200
300
400
500

0

FLOW RATE
OF PERMEATION

# FIG. 8A

# FIG. 8B

## INTERNATIONAL SEARCH REPORT

| | International application No. |
|---|---|
| | **PCT/JP2024/020228** |

### A. CLASSIFICATION OF SUBJECT MATTER

*C12P 1/00*(2006.01)i; *B01D 63/02*(2006.01)i; *C12M 1/00*(2006.01)i; *C12M 3/06*(2006.01)i; *C12N 1/02*(2006.01)i; *C12P 21/08*(2006.01)i

FI: C12P1/00 Z; C12M1/00 D; C12M3/06; C12P21/08; B01D63/02; C12N1/02

According to International Patent Classification (IPC) or to both national classification and IPC

### B. FIELDS SEARCHED

Minimum documentation searched (classification system followed by classification symbols)

C12P1/00; B01D63/02; C12M1/00; C12M3/06; C12N1/02; C12P21/08; B01D63/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2012/090863 A1 (TORAY INDUSTRIES, INC.) 05 July 2012 (2012-07-05) reference example 3 | 13-20 |
| A | entire text | 1-12 |
| A | JP 2011-16116 A (ASAHI KASEI CHEMICALS CORPORATION) 27 January 2011 (2011-01-27) entire text | 1-20 |
| A | WO 2009/054226 A1 (ASAHI KASEI CHEMICALS CORPORATION) 30 April 2009 (2009-04-30) entire text | 1-20 |
| A | JP 2023-33334 A (TORAY INDUSTRIES, INC.) 10 March 2023 (2023-03-10) entire text | 1-20 |
| P, A | WO 2024/024337 A1 (DAICEL CORP.) 01 February 2024 (2024-02-01) entire text | 1-20 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **22 July 2024** | **06 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

| International application No. |
| --- |
| **PCT/JP2024/020228** |

| C. | DOCUMENTS CONSIDERED TO BE RELEVANT | |
| --- | --- | --- |
| Category* | Citation of document, with indication, where appropriate, of the relevant passages | Relevant to claim No. |
| P, A | WO 2024/106442 A1 (ASAHI KASEI MEDICAL CO., LTD.) 23 May 2024 (2024-05-23) entire text | 1-20 |

Form PCT/ISA/210 (second sheet) (July 2022)

## INTERNATIONAL SEARCH REPORT
### Information on patent family members

| International application No. |
|---|
| **PCT/JP2024/020228** |

| Patent document cited in search report | | | Publication date (day/month/year) | Patent family member(s) | | | Publication date (day/month/year) |
|---|---|---|---|---|---|---|---|
| WO | 2012/090863 | A1 | 05 July 2012 | US | 2013/0280767 | A1 | |
| | | | | reference example 3 | | | |
| | | | | EP | 2659957 | A1 | |
| | | | | CN | 103282109 | A | |
| | | | | KR | 10-2014-0032949 | A | |
| | | | | TW | 201231153 | A | |
| JP | 2011-16116 | A | 27 January 2011 | (Family: none) | | | |
| WO | 2009/054226 | A1 | 30 April 2009 | US | 2010/0228010 | A1 | |
| | | | | entire text | | | |
| | | | | US | 2012/0322983 | A1 | |
| | | | | EP | 2226331 | A1 | |
| | | | | EP | 2719705 | A1 | |
| | | | | AU | 2008315124 | A | |
| | | | | KR | 10-2010-0043243 | A | |
| | | | | CA | 2703086 | A | |
| | | | | CN | 101835791 | A | |
| | | | | TW | 200932330 | A | |
| | | | | TW | 201350183 | A | |
| JP | 2023-33334 | A | 10 March 2023 | US | 2023/0256394 | A1 | |
| | | | | entire text | | | |
| | | | | EP | 4173698 | A1 | |
| | | | | CN | 115996788 | A | |
| WO | 2024/024337 | A1 | 01 February 2024 | (Family: none) | | | |
| WO | 2024/106442 | A1 | 23 May 2024 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021511202 A
- JP 2018183150 A
- JP 2000517188 A
- JP 2023095894 A